# EUROPEAN PATENT SPECIFICATION

(11) **EP 2 082 044 B1**
(45) Date of publication and mention of the grant of the patent: **01.06.2016**
(21) Application number: 07821517.5
(22) Date of filing: 18.10.2007
(51) Int. Cl.: C12N 15/67, C12N 15/77

(54) **METHOD OF INCREASING GENE EXPRESSION USING MODIFIED CODON USAGE**
VERFAHREN ZUR ERHÖHUNG DER GENEXPRESSION MITTELS MODIFIZIERTER CODONVERWENDUNG
PROCÉDÉ PERMETTANT D'AUGMENTER L'EXPRESSION GÉNIQUE PAR UNE UTILISATION DE CODONS MODIFIÉES

(30) Priority: 24.10.2006 EP 06122868
(43) Date of publication of application: 29.07.2009
(73) Proprietor: BASF SE, 67056 Ludwigshafen am Rhein (DE)
(72) Inventor: HEROLD, Andrea, 68775 Ketsch (DE); KLOPPROGGE, Corinna, 68239 Mannheim (DE); SCHRÖDER, Hartwig, 69226 Nussloch (DE); ZELDER, Oskar, 67346 Speyer (DE); JEONG, Weol Kyu, Gunsan 573-730 (KR)
(74) Representative: BASF IP Association
(86) International application number: PCT/EP2007/061152
(87) International publication number: WO 2008/049782

(56) References cited:
- EP-A1- 1 302 537
- EP-A2- 1 055 725
- WO-A-02/10209
- WO-A-96/09378
- WO-A1-02/12504
- WO-A2-2006/008098
- GUSTAFSSON C ET AL: "Codon bias and heterologous protein expression" TRENDS IN BIOTECHNOLOGY, ELSEVIER PUBLICATIONS, CAMBRIDGE, GB, vol. 22, no. 7, July 2004 (2004-07), pages 346-353, XP004520507 ISSN: 0167-7799
- SHARP P M ET AL: "THE CODON ADAPTATION INDEX - A MEASURE OF DIRECTIONAL SYNONYMOUS CODON USAGE BIAS, AND ITS POTENTIAL APPLICATIONS" NUCLEIC ACIDS RESEARCH, OXFORD UNIVERSITY PRESS, SURREY, GB, vol. 15, no. 3, 11 February 1987 (1987-02-11), pages 1281-1295, XP001122356 ISSN: 0305-1048
- DATABASE EMBL [Online] 8 July 1999 (1999-07-08), "Clostridium subterminale L-lysine 2,3-aminomutase (kamA) gene, complete cds." XP002470801 retrieved from EBI accession no. EMBL:AF159146 Database accession no. AF159146
- YEH P ET AL: "NUCLEOTIDE SEQUENCE OF THE LYSA GENE OF CORYNEBACTERIUM GLUTAMICUM AND POSSIBLE MECHANISMS FOR MODULATION OF ITS EXPRESSION" MOLECULAR AND GENERAL GENETICS, SPRINGER VERLAG, BERLIN, DE, vol. 212, no. 1, 1988, pages 112-119, XP001002987 ISSN: 0026-8925
- CONNELL N D: "Expression systems for use in actinomycetes and related organisms" CURRENT OPINION IN BIOTECHNOLOGY 01 OCT 2001 UNITED KINGDOM, vol. 12, no. 5, 1 October 2001 (2001-10-01), pages 446-449, XP002470819 ISSN: 0958-1669
- EIKMANNS B J: "IDENTIFICATION, SEQUENCE ANALYSIS, AND EXPRESSION OF A CORYNEBACTERIUM GLUTAMICUM GENE CLUSTER ENCODING THE THREE GLYCOLYTIC ENZYMES GLYCERALDEHYDE-3-PHOSPHATE DEHYDROGENASE, 3-PHOSPHOGLYCERATE KINASE, AND TRIOSEPHOSPHATE ISOMERASE", JOURNAL OF BACTERIOLOGY, AMERICAN SOCIETY FOR MICROBIOLOGY, WASHINGTON, DC; US, vol. 174, no. 19, 1 October 1992 (1992-10-01), pages 6076-6086, XP000979491, ISSN: 0021-9193
- GLICK B R ET AL: "FACTORS AFFECTING THE EXPRESSION OF FOREIGN PROTEINS IN ESCHERICHIA-COLI", JOURNAL OF INDUSTRIAL MICROBIOLOGY, vol. 1, no. 5, 1987, pages 277-282, ISSN: 0169-4146

### SEQUENCE LISTING

<110> BASF AG
<120> Method of increasing gene expression using modified codon usage
<130> B 8440 / DB
<150> EP06122868.0
   <151> 2006-10-24
<160> 19
<170> PatentIn version 3.3
<210> 1
   <211> 1251
   <212> DNA
   <213> Artificial
<220>
   <223> Synthetic gene sequence of Lysine-2,3-aminomutase
<400> 1
   atgatcaacc gccgctacga actgttcaag gatgtgtccg atgcagattg gaacgattgg     60
   cgctggcagg tgcgcaaccg catcgaaacc gtggaagaac tgaagaagta catcccactg    120
   accaaggaag aagaagaagg cgtggcacag tgcgtgaagt ccctgcgcat ggcaatcacc    180
   ccatactacc tgtccctgat cgatccaaac gatccaaacg atccagtgcg caagcaggca    240
   atcccaaccg cactggaact gaacaaggca gcagcagatc tggaagatcc actgcacgaa    300
   gataccgatt ccccagtgcc aggcctgacc caccgctacc cagatcgcgt gctgctgctg    360
   atcaccgata tgtgctccat gtactgccgc cactgcaccc gccgccgctt cgcaggccag    420
   tccgatgatt ccatgccaat ggaacgcatc gataaggcaa tcgattacat ccgcaacacc    480
   ccacaggtgc gcgatgtgct gctgtccggc ggcgatgcac tgctggtgtc cgatgaaacc    540
   ctggaataca tcatcgcaaa gctgcgcgaa atcccacacg tggaaatcgt gcgcatcggc    600
   tcccgcaccc cagtggtgct gccacagcgc atcaccccag aactggtgaa catgctgaag     660
   aagtaccacc cagtgtggct gaacacccac ttcaaccacc caaacgaaat caccgaagaa    720
   tccacccgcg catgccagct gctggcagat gcaggcgtgc cactgggcaa ccagtccgtg    780
   ctgctgcgcg gcgtgaacga ttgcgtgcac gtgatgaagg aactggtgaa caagctggtg    840
   aagatccgcg tgcgcccata ctacatctac cagtgcgatc tgtccctggg cctggaacac    900
   ttccgcaccc cagtgtccaa gggcatcgaa atcatcgaag gcctgcgcgg ccacacctcc    960
   ggctactgcg tgccaacctt cgtggtggat gcaccaggcg gcggcggcaa gaccccagtg   1020
   atgccaaact acgtgatctc ccagtcccac gataaggtga tcctgcgcaa cttcgaaggc   1080
   gtgatcacca cctactccga accaatcaac tacaccccag gctgcaactg cgatgtgtgc   1140
   accggcaaga agaaggtgca caaggtgggc gtggcaggcc tgctgaacgg cgaaggcatg   1200
   gcactggaac cagtgggcct ggaacgcaac aagcgccacg tgcaggaata a            1251
<210> 2
   <211> 1521
   <212> DNA
   <213> Artificial
<220>
   <223> Vector Insert comprising gene sequence of synthetic Lysine-2,3-aminomutase pClik 5a MCS
<400> 2
   atgactagtt agctgccaat tattccgggc ttgtgacccg ctacccgata aataggtcgg 60
   ctgaaaaatt tcgttgcaat atcaacaaaa aggcctatca ttgggaggtg tcgcaccaag 120
   tacttttgcg aagcgccatc tgacggattt tcaaaagatg tatatgctcg gtgcggaaac 180
   ctacgaaagg atttttaccc catgatcaac cgccgctacg aactgttcaa ggatgtgtcc 240
   gatgcagatt ggaacgattg gcgctggcag gtgcgcaacc gcatcgaaac cgtggaagaa 300
   ctgaagaagt acatcccact gaccaaggaa gaagaagaag gcgtggcaca gtgcgtgaag 360
   tccctgcgca tggcaatcac cccatactac ctgtccctga tcgatccaaa cgatccaaac 420
   gatccagtgc gcaagcaggc aatcccaacc gcactggaac tgaacaaggc agcagcagat 480
   ctggaagatc cactgcacga agataccgat tccccagtgc caggcctgac ccaccgctac 540
   ccagatcgcg tgctgctgct gatcaccgat atgtgctcca tgtactgccg ccactgcacc 600
   cgccgccgct tcgcaggcca gtccgatgat tccatgccaa tggaacgcat cgataaggca 660
   atcgattaca tccgcaacac cccacaggtg cgcgatgtgc tgctgtccgg cggcgatgca 720
   ctgctggtgt ccgatgaaac cctggaatac atcatcgcaa agctgcgcga aatcccacac 780
   gtggaaatcg tgcgcatcgg ctcccgcacc ccagtggtgc tgccacagcg catcacccca 840
   gaactggtga acatgctgaa gaagtaccac ccagtgtggc tgaacaccca cttcaaccac 900
   ccaaacgaaa tcaccgaaga atccacccgc gcatgccagc tgctggcaga tgcaggcgtg 960
   ccactgggca accagtccgt gctgctgcgc ggcgtgaacg attgcgtgca cgtgatgaag 1020
   gaactggtga acaagctggt gaagatccgc gtgcgcccat actacatcta ccagtgcgat 1080
   ctgtccctgg gcctggaaca cttccgcacc ccagtgtcca agggcatcga aatcatcgaa 1140
   ggcctgcgcg gccacacctc cggctactgc gtgccaacct tcgtggtgga tgcaccaggc 1200
   ggcggcggca agaccccagt gatgccaaac tacgtgatct cccagtccca cgataaggtg 1260
   atcctgcgca acttcgaagg cgtgatcacc acctactccg aaccaatcaa ctacacccca 1320
   ggctgcaact gcgatgtgtg caccggcaag aagaaggtgc acaaggtggg cgtggcaggc 1380
   ctgctgaacg gcgaaggcat ggcactggaa ccagtgggcc tggaacgcaa caagcgccac 1440
   gtgcaggaat aaagttctgt gaaaaacacc gtggggcagt ttctgcttcg cggtgttttt 1500
   tatttgtggg gcaactagtt c 1521
<210> 3
   <211> 17
   <212> DNA
   <213> Artificial
<220>
   <223> Primer WKJ90
<400> 3
   cctaacacag aaatgtc 17
<210> 4
   <211> 20
   <212> DNA
   <213> Artificial
<220>
   <223> Primer WKJ65
<400> 4
   cagtctgcat cgctaacatc 20
<210> 5
   <211> 40
   <212> DNA
   <213> Artificial
<220>
   <223> Primer WKJ105
<400> 5
   atcttcttgg cagaactcat gggtaaaaaa tcctttcgta 40
<210> 6
   <211> 34
   <212> DNA
   <213> Artificial
<220>
   <223> Primer WKJ106
<400> 6
   gagagagatc tagatagctg ccaattattc cggg 34
<210> 7
   <211> 6601
   <212> DNA
   <213> Artificial
<220>
   <223> Plasmid pClik 5a MCS Psod genom KamA
<400> 7
   tcgatttaaa tctcgagagg cctgacgtcg ggcccggtac cacgcgtcat atgactagtt 60
   agctgccaat tattccgggc ttgtgacccg ctacccgata aataggtcgg ctgaaaaatt 120
   tcgttgcaat atcaacaaaa aggcctatca ttgggaggtg tcgcaccaag tacttttgcg 180
   aagcgccatc tgacggattt tcaaaagatg tatatgctcg gtgcggaaac ctacgaaagg 240
   atttttaccc catgataaat agaagatatg aattatttaa agatgttagc gatgcagact 300
   ggaatgactg gagatggcaa gtaagaaaca gaatagaaac tgttgaagaa ctaaagaaat 360
   acataccatt aacaaaagaa gaagaagaag gagtagctca atgtgtaaaa tcattaagaa 420
   tggctattac tccatattat ctatcattaa tcgatcctaa cgatcctaat gatccagtaa 480
   gaaaacaagc tattccaaca gcattagagc ttaacaaagc tgctgcagat cttgaagacc 540
   cattacatga agatacagat tcaccagtac ctggattaac tcacagatat ccagatagag 600
   tattattatt aataactgat atgtgctcaa tgtactgcag acactgtaca agaagaagat 660
   ttgcaggaca aagcgatgac tctatgccaa tggaaagaat agataaagct atagattata 720
   tcagaaatac tcctcaagtt agagacgtat tattatcagg tggagacgct cttttagtat 780
   ctgatgaaac attagaatac atcatagcta aattaagaga aataccacac gttgaaatag 840
   taagaatagg ttcaagaact ccagttgttc ttccacaaag aataactcca gaacttgtaa 900
   atatgcttaa aaaatatcat ccagtatggt taaacactca ctttaaccat ccaaatgaaa 960
   taacagaaga atcaactaga gcttgtcaat tacttgctga cgcaggagta cctctaggaa 1020
   accaatcagt tttattaaga ggagttaacg attgcgtaca cgtaatgaaa gaattagtta 1080
   acaaattagt aaaaataaga gtaagacctt actacatcta tcaatgtgac ttatcattag 1140
   gacttgagca cttcagaact ccagtttcta aaggtatcga aatcattgaa ggattaagag 1200
   gacatacttc aggatactgc gtaccaacat tcgttgttga cgctccaggt ggtggtggaa 1260
   aaacaccagt tatgccaaac tacgttattt cacaaagtca tgacaaagta atattaagaa 1320
   actttgaagg tgttataaca acttattcag aaccaataaa ctatactcca ggatgcaact 1380
   gtgatgtttg cactggcaag aaaaaagttc ataaggttgg agttgctgga ttattaaacg 1440
   gagaaggaat ggctctagaa ccagtaggat tagagagaaa taagagacac gttcaagaat 1500
   aaagttctgt gaaaaacacc gtggggcagt ttctgcttcg cggtgttttt tatttgtggg 1560
   gcaactagtt cggacctagg gatatcgtcg acatcgatgc tcttctgcgt taattaacaa 1620
   ttgggatcct ctagacccgg gatttaaatc gctagcgggc tgctaaagga agcggaacac 1680
   gtagaaagcc agtccgcaga aacggtgctg accccggatg aatgtcagct actgggctat 1740
   ctggacaagg gaaaacgcaa gcgcaaagag aaagcaggta gcttgcagtg ggcttacatg 1800
   gcgatagcta gactgggcgg ttttatggac agcaagcgaa ccggaattgc cagctggggc 1860
   gccctctggt aaggttggga agccctgcaa agtaaactgg atggctttct tgccgccaag 1920
   gatctgatgg cgcaggggat caagatctga tcaagagaca ggatgaggat cgtttcgcat 1980
   gattgaacaa gatggattgc acgcaggttc tccggccgct tgggtggaga ggctattcgg 2040
   ctatgactgg gcacaacaga caatcggctg ctctgatgcc gccgtgttcc ggctgtcagc 2100
   gcaggggcgc ccggttcttt ttgtcaagac cgacctgtcc ggtgccctga atgaactgca 2160
   ggacgaggca gcgcggctat cgtggctggc cacgacgggc gttccttgcg cagctgtgct 2220
   cgacgttgtc actgaagcgg gaagggactg gctgctattg ggcgaagtgc cggggcagga 2280
   tctcctgtca tctcaccttg ctcctgccga gaaagtatcc atcatggctg atgcaatgcg 2340
   gcggctgcat acgcttgatc cggctacctg cccattcgac caccaagcga aacatcgcat 2400
   cgagcgagca cgtactcgga tggaagccgg tcttgtcgat caggatgatc tggacgaaga 2460
   gcatcagggg ctcgcgccag ccgaactgtt cgccaggctc aaggcgcgca tgcccgacgg 2520
   cgaggatctc gtcgtgaccc atggcgatgc ctgcttgccg aatatcatgg tggaaaatgg 2580
   ccgcttttct ggattcatcg actgtggccg gctgggtgtg gcggaccgct atcaggacat 2640
   agcgttggct acccgtgata ttgctgaaga gcttggcggc gaatgggctg accgcttcct 2700
   cgtgctttac ggtatcgccg ctcccgattc gcagcgcatc gccttctatc gccttcttga 2760
   cgagttcttc tgagcgggac tctggggttc gaaatgaccg accaagcgac gcccaacctg 2820
   ccatcacgag atttcgattc caccgccgcc ttctatgaaa ggttgggctt cggaatcgtt 2880
   ttccgggacg ccggctggat gatcctccag cgcggggatc tcatgctgga gttcttcgcc 2940
   cacgctagcg gcgcgccggc cggcccggtg tgaaataccg cacagatgcg taaggagaaa 3000
   ataccgcatc aggcgctctt ccgcttcctc gctcactgac tcgctgcgct cggtcgttcg 3060
   gctgcggcga gcggtatcag ctcactcaaa ggcggtaata cggttatcca cagaatcagg 3120
   ggataacgca ggaaagaaca tgtgagcaaa aggccagcaa aaggccagga accgtaaaaa 3180
   ggccgcgttg ctggcgtttt tccataggct ccgcccccct gacgagcatc acaaaaatcg 3240
   acgctcaagt cagaggtggc gaaacccgac aggactataa agataccagg cgtttccccc 3300
   tggaagctcc ctcgtgcgct ctcctgttcc gaccctgccg cttaccggat acctgtccgc 3360
   ctttctccct tcgggaagcg tggcgctttc tcatagctca cgctgtaggt atctcagttc 3420
   ggtgtaggtc gttcgctcca agctgggctg tgtgcacgaa ccccccgttc agcccgaccg 3480
   ctgcgcctta tccggtaact atcgtcttga gtccaacccg gtaagacacg acttatcgcc 3540
   actggcagca gccactggta acaggattag cagagcgagg tatgtaggcg gtgctacaga 3600
   gttcttgaag tggtggccta actacggcta cactagaagg acagtatttg gtatctgcgc 3660
   tctgctgaag ccagttacct tcggaaaaag agttggtagc tcttgatccg gcaaacaaac 3720
   caccgctggt agcggtggtt tttttgtttg caagcagcag attacgcgca gaaaaaaagg 3780
   atctcaagaa gatcctttga tcttttctac ggggtctgac gctcagtgga acgaaaactc 3840
   acgttaaggg attttggtca tgagattatc aaaaaggatc ttcacctaga tccttttaaa 3900
   ggccggccgc ggccgcgcaa agtcccgctt cgtgaaaatt ttcgtgccgc gtgattttcc 3960
   gccaaaaact ttaacgaacg ttcgttataa tggtgtcatg accttcacga cgaagtacta 4020
   aaattggccc gaatcatcag ctatggatct ctctgatgtc gcgctggagt ccgacgcgct 4080
   cgatgctgcc gtcgatttaa aaacggtgat cggatttttc cgagctctcg atacgacgga 4140
   cgcgccagca tcacgagact gggccagtgc cgcgagcgac ctagaaactc tcgtggcgga 4200
   tcttgaggag ctggctgacg agctgcgtgc tcggccagcg ccaggaggac gcacagtagt 4260
   ggaggatgca atcagttgcg cctactgcgg tggcctgatt cctccccggc ctgacccgcg 4320
   aggacggcgc gcaaaatatt gctcagatgc gtgtcgtgcc gcagccagcc gcgagcgcgc 4380
   caacaaacgc cacgccgagg agctggaggc ggctaggtcg caaatggcgc tggaagtgcg 4440
   tcccccgagc gaaattttgg ccatggtcgt cacagagctg gaagcggcag cgagaattat 4500
   cgcgatcgtg gcggtgcccg caggcatgac aaacatcgta aatgccgcgt ttcgtgtgcc 4560
   gtggccgccc aggacgtgtc agcgccgcca ccacctgcac cgaatcggca gcagcgtcgc 4620
   gcgtcgaaaa agcgcacagg cggcaagaag cgataagctg cacgaatacc tgaaaaatgt 4680
   tgaacgcccc gtgagcggta actcacaggg cgtcggctaa cccccagtcc aaacctggga 4740
   gaaagcgctc aaaaatgact ctagcggatt cacgagacat tgacacaccg gcctggaaat 4800
   tttccgctga tctgttcgac acccatcccg agctcgcgct gcgatcacgt ggctggacga 4860
   gcgaagaccg ccgcgaattc ctcgctcacc tgggcagaga aaatttccag ggcagcaaga 4920
   cccgcgactt cgccagcgct tggatcaaag acccggacac ggagaaacac agccgaagtt 4980
   ataccgagtt ggttcaaaat cgcttgcccg gtgccagtat gttgctctga cgcacgcgca 5040
   gcacgcagcc gtgcttgtcc tggacattga tgtgccgagc caccaggccg gcgggaaaat 5100
   cgagcacgta aaccccgagg tctacgcgat tttggagcgc tgggcacgcc tggaaaaagc 5160
   gccagcttgg atcggcgtga atccactgag cgggaaatgc cagctcatct ggctcattga 5220
   tccggtgtat gccgcagcag gcatgagcag cccgaatatg cgcctgctgg ctgcaacgac 5280
   cgaggaaatg acccgcgttt tcggcgctga ccaggctttt tcacataggc tgagccgtgg 5340
   ccactgcact ctccgacgat cccagccgta ccgctggcat gcccagcaca atcgcgtgga 5400
   tcgcctagct gatcttatgg aggttgctcg catgatctca ggcacagaaa aacctaaaaa 5460
   acgctatgag caggagtttt ctagcggacg ggcacgtatc gaagcggcaa gaaaagccac 5520
   tgcggaagca aaagcacttg ccacgcttga agcaagcctg ccgagcgccg ctgaagcgtc 5580
   tggagagctg atcgacggcg tccgtgtcct ctggactgct ccagggcgtg ccgcccgtga 5640
   tgagacggct tttcgccacg ctttgactgt gggataccag ttaaaagcgg ctggtgagcg 5700
   cctaaaagac accaagggtc atcgagccta cgagcgtgcc tacaccgtcg ctcaggcggt 5760
   cggaggaggc cgtgagcctg atctgccgcc ggactgtgac cgccagacgg attggccgcg 5820
   acgtgtgcgc ggctacgtcg ctaaaggcca gccagtcgtc cctgctcgtc agacagagac 5880
   gcagagccag ccgaggcgaa aagctctggc cactatggga agacgtggcg gtaaaaaggc 5940
   cgcagaacgc tggaaagacc caaacagtga gtacgcccga gcacagcgag aaaaactagc 6000
   taagtccagt caacgacaag ctaggaaagc taaaggaaat cgcttgacca ttgcaggttg 6060
   gtttatgact gttgagggag agactggctc gtggccgaca atcaatgaag ctatgtctga 6120
   atttagcgtg tcacgtcaga ccgtgaatag agcacttaag gtctgcgggc attgaacttc 6180
   cacgaggacg ccgaaagctt cccagtaaat gtgccatctc gtaggcagaa aacggttccc 6240
   ccgtagggtc tctctcttgg cctcctttct aggtcgggct gattgctctt gaagctctct 6300
   aggggggctc acaccatagg cagataacgt tccccaccgg ctcgcctcgt aagcgcacaa 6360
   ggactgctcc caaagatctt caaagccact gccgcgactg ccttcgcgaa gccttgcccc 6420
   gcggaaattt cctccaccga gttcgtgcac acccctatgc caagcttctt tcaccctaaa 6480
   ttcgagagat tggattctta ccgtggaaat tcttcgcaaa aatcgtcccc tgatcgccct 6540
   tgcgacgttg gcgtcggtgc cgctggttgc gcttggcttg accgacttga tcagcggccg 6600
   c 6601
<210> 8
   <211> 1338
   <212> DNA
   <213> Artificial
<220>
   <223> synthetic lysA sequence
<400> 8
   atggctaccg ttgaaaactt caacgaactt cccgcacacg tgtggccacg caatgccgtg      60
   cgccaggaag atggcgtggt gaccgtggct ggtgtgcctc tgcctgatct ggctgaagaa     120
   tacggaaccc cactgttcgt ggtggacgaa gacgatttcc gttcccgctg ccgcgacatg     180
   gctaccgcat tcggtggccc aggcaatgtg cactacgcat ccaaggcatt cctgaccaag     240
   accatcgcac gctgggtgga tgaagaaggc ctggcactgg atatcgcatc catcaacgaa     300
   ctgggcatcg cactggcagc aggcttccca gcatcccgca tcaccgcaca cggcaacaac     360
   aagggcgtgg aattcctgcg cgcactggtg cagaacggcg tgggccacgt ggtgctggat     420
   tccgcacagg aactggaact gctggattac gtggcagcag gcgaaggcaa gatccaggat     480
   gtgctgatcc gcgtgaagcc aggcatcgaa gcacacaccc acgaattcat cgcaacctcc     540
   cacgaagatc agaagttcgg cttctccctg gcatccggct ccgcattcga agcagcaaag     600
   gcagcaaaca acgcagaaaa cctgaacctg gtgggcctgc actgccacgt gggctcccag     660
   gtgttcgatg cagaaggctt caagctggca gcagaacgcg tgctgggcct gtactcccag     720
   atccactccg aactgggcgt ggcactgcca gaactggatc tgggcggcgg ctacggcatc     780
   gcatacaccg cagcagaaga accactgaac gtggcagaag tggcatccga tctgctgacc     840
   gcagtgggca agatggcagc ggaactaggc atcgatgcac caaccgtgct tgttgagccc     900
   ggccgcgcta tcgcaggccc ctccaccgtg accatctacg aagtcggcac caccaaagac     960
   gtccacgtag acgacgacaa aacccgccgt tacatcgcag tggatggcgg catgtccgat    1020
   aacatccgcc cagcactgta cggctccgaa tacgacgccc gcgtagtatc ccgcttcgca    1080
   gaaggcgatc cagtgtccac ccgcatcgtg ggctcccact gcgaatccgg cgatatcctg    1140
   atcaacgatg aaatctaccc atccgatatc acctccggcg atttcctggc actggcagca    1200
   accggcgcat actgctacgc aatgtcctcc cgctacaacg cattcacccg cccagcagtg    1260
   gtgtccgtgc gcgcaggctc ctcccgcctg atgctgcgcc gcgaaaccct ggatgatatc    1320
   ctgtccctgg aagcataa                                                  1338
<210> 9
   <211> 2560
   <212> DNA
   <213> Artificial
<220>
   <223> synthetic lysA sequence including up- and downstream regions
<400> 9
   actatgacgt cggcgttgaa gtcctgattg gccagatggt gaacctgctt cgcgacggca 60
   aggcagtgcg tatgtccaag cgtgcaggca ccgtggtcac cctagatgac ctcgttgaag 120
   caatcggcat cgatgcggcg cgttactccc tgatccgttc ctccgtggat tcttccctgg 180
   atatcgatct cggcctgtgg gaatcccagt cctccgacaa ccctgtgtac tacgtgcagt 240
   acggacacgc tcgtctgtgc tccatcgcgc gcaaggcaga gaccttgggt gtcaccgagg 300
   aaggcgcaga cctatctcta ctgacccacg accgcgaagg cgatctcatc cgcacactcg 360
   gagagttccc agcagtggtg aaggctgccg ctgacctacg tgaaccacac cgcattgccc 420
   gctatgctga ggaattagct ggaactttcc accgcttcta cgattcctgc cacatccttc 480
   caaaggttga tgaggatacg gcaccaatcc acacagcacg tctggcactt gcagcagcaa 540
   cccgccagac cctcgctaac gccctgcacc tggttggcgt ttccgcaccg gagaagatgt 600
   aacaatggct accgttgaaa acttcaacga acttcccgca cacgtgtggc cacgcaatgc 660
   cgtgcgccag gaagatggcg tggtgaccgt ggctggtgtg cctctgcctg atctggctga 720
   agaatacgga accccactgt tcgtggtgga cgaagacgat ttccgttccc gctgccgcga 780
   catggctacc gcattcggtg gcccaggcaa tgtgcactac gcatccaagg cattcctgac 840
   caagaccatc gcacgctggg tggatgaaga aggcctggca ctggatatcg catccatcaa 900
   cgaactgggc atcgcactgg cagcaggctt cccagcatcc cgcatcaccg cacacggcaa 960
   caacaagggc gtggaattcc tgcgcgcact ggtgcagaac ggcgtgggcc acgtggtgct 1020
   ggattccgca caggaactgg aactgctgga ttacgtggca gcaggcgaag gcaagatcca 1080
   ggatgtgctg atccgcgtga agccaggcat cgaagcacac acccacgaat tcatcgcaac 1140
   ctcccacgaa gatcagaagt tcggcttctc cctggcatcc ggctccgcat tcgaagcagc 1200
   aaaggcagca aacaacgcag aaaacctgaa cctggtgggc ctgcactgcc acgtgggctc 1260
   ccaggtgttc gatgcagaag gcttcaagct ggcagcagaa cgcgtgctgg gcctgtactc 1320
   ccagatccac tccgaactgg gcgtggcact gccagaactg gatctgggcg gcggctacgg 1380
   catcgcatac accgcagcag aagaaccact gaacgtggca gaagtggcat ccgatctgct 1440
   gaccgcagtg ggcaagatgg cagcggaact aggcatcgat gcaccaaccg tgcttgttga 1500
   gcccggccgc gctatcgcag gcccctccac cgtgaccatc tacgaagtcg gcaccaccaa 1560
   agacgtccac gtagacgacg acaaaacccg ccgttacatc gcagtggatg gcggcatgtc 1620
   cgataacatc cgcccagcac tgtacggctc cgaatacgac gcccgcgtag tatcccgctt 1680
   cgcagaaggc gatccagtgt ccacccgcat cgtgggctcc cactgcgaat ccggcgatat 1740
   cctgatcaac gatgaaatct acccatccga tatcacctcc ggcgatttcc tggcactggc 1800
   agcaaccggc gcatactgct acgcaatgtc ctcccgctac aacgcattca cccgcccagc 1860
   agtggtgtcc gtgcgcgcag gctcctcccg cctgatgctg cgccgcgaaa ccctggatga 1920
   tatcctgtcc ctggaagcat aacgcttttc gacgcctgac cccgcccttc accttcgccg 1980
   tggagggcgg ttttgggttt ctgggtttct ttgcgaactt aaggtgtcta ggggtctgca 2040
   gtcgaggctt cagaggtttt attgctcgac ttcacgtgtg ctgacttcgc gtgctcaact 2100
   tctcatgagg cgacatcagt cgaaaagccg gtcattgtcg tatgaagtct gcgcacgaga 2160
   agttgagcgg aagaagtcgg tgttaagaag tcgaccgcgt gaagtcgccc tttaggagaa 2220
   ttctgactaa ctggagccaa aacttgatcc actcgagagc tgtgcagtct ctttttcctt 2280
   caattctgcc tgctcgagct cgtagaagta gaggtctact tcagttggtt caccttgcac 2340
   acaagcatga agtagtgggt aggtcgagtt gttaaatgcg gtgtagaagg ggagtagttc 2400
   gctagcaaag gttaatttgg agtcgctgta ctgcgggttc tcgggtggag tattcccgga 2460
   ggattcaaga aatcttgacg catctttgat gaggtatgtt tggaattcgt cggcaccttc 2520
   ctcgccggag aggtagtagg agttgtcgtc tagaagtagt 2560
<210> 10
   <211> 3666
   <212> DNA
   <213> Corynebacterium glutamicum
<400> 10
   atgtcgactt cagttacttc accagcccac aacaacgcac attcctccga atttttggat 60
   gcgttggcaa accatgtgtt gatcggcgac ggcgccatgg gcacccagct ccaaggcttt 120
   gacctggacg tggaaaagga tttccttgat ctggaggggt gtaatgagat tctcaacgac 180
   acccgccctg atgtgttgag gcagattcac cgcgcctact ttgaggcggg agctgacttg 240
   gttgagacca atacttttgg ttgcaacctg ccgaacttgg cggattatga catcgctgat 300
   cgttgccgtg agcttgccta caagggcact gcagtggcta gggaagtggc tgatgagatg 360
   gggccgggcc gaaacggcat gcggcgtttc gtggttggtt ccctgggacc tggaacgaag 420
   cttccatcgc tgggccatgc accgtatgca gatttgcgtg ggcactacaa ggaagcagcg 480
   cttggcatca tcgacggtgg tggcgatgcc tttttgattg agactgctca ggacttgctt 540
   caggtcaagg ctgcggttca cggcgttcaa gatgccatgg ctgaacttga tacattcttg 600
   cccattattt gccacgtcac cgtagagacc accggcacca tgctcatggg ttctgagatc 660
   ggtgccgcgt tgacagcgct gcagccactg ggtatcgaca tgattggtct gaactgcgcc 720
   accggcccag atgagatgag cgagcacctg cgttacctgt ccaagcacgc cgatattcct 780
   gtgtcggtga tgcctaacgc aggtcttcct gtcctgggta aaaacggtgc agaataccca 840
   cttgaggctg aggatttggc gcaggcgctg gctggattcg tctccgaata tggcctgtcc 900
   atggtgggtg gttgttgtgg caccacacct gagcacatcc gtgcggtccg cgatgcggtg 960
   gttggtgttc cagagcagga aacctccaca ctgaccaaga tccctgcagg ccctgttgag 1020
   caggcctccc gcgaggtgga gaaagaggac tccgtcgcgt cgctgtacac ctcggtgcca 1080
   ttgtcccagg aaaccggcat ttccatgatc ggtgagcgca ccaactccaa cggttccaag 1140
   gcattccgtg aggcaatgct gtctggcgat tgggaaaagt gtgtggatat tgccaagcag 1200
   caaacccgcg atggtgcaca catgctggat ctttgtgtgg attacgtggg acgagacggc 1260
   accgccgata tggcgacctt ggcagcactt cttgctacca gctccacttt gccaatcatg 1320
   attgactcca ccgagccaga ggttattcgc acaggccttg agcacttggg tggacgaagc 1380
   atcgttaact ccgtcaactt tgaagacggc gatggccctg agtcccgcta ccagcgcatc 1440
   atgaaactgg taaagcagca cggtgcggcc gtggttgcgc tgaccattga tgaggaaggc 1500
   caggcacgta ccgctgagca caaggtgcgc attgctaaac gactgattga cgatatcacc 1560
   ggcagctacg gcctggatat caaagacatc gttgtggact gcctgacctt cccgatctct 1620
   actggccagg aagaaaccag gcgagatggc attgaaacca tcgaagccat ccgcgagctg 1680
   aagaagctct acccagaaat ccacaccacc ctgggtctgt ccaatatttc cttcggcctg 1740
   aaccctgctg cacgccaggt tcttaactct gtgttcctca atgagtgcat tgaggctggt 1800
   ctggactctg cgattgcgca cagctccaag attttgccga tgaaccgcat tgatgatcgc 1860
   cagcgcgaag tggcgttgga tatggtctat gatcgccgca ccgaggatta cgatccgctg 1920
   caggaattca tgcagctgtt tgagggcgtt tctgctgccg atgccaagga tgctcgcgct 1980
   gaacagctgg ccgctatgcc tttgtttgag cgtttggcac agcgcatcat cgacggcgat 2040
   aagaatggcc ttgaggatga tctggaagca ggcatgaagg agaagtctcc tattgcgatc 2100
   atcaacgagg accttctcaa cggcatgaag accgtgggtg agctgtttgg ttccggacag 2160
   atgcagctgc cattcgtgct gcaatcggca gaaaccatga aaactgcggt ggcctatttg 2220
   gaaccgttca tggaagagga agcagaagct accggatctg cgcaggcaga gggcaagggc 2280
   aaaatcgtcg tggccaccgt caagggtgac gtgcacgata tcggcaagaa cttggtggac 2340
   atcattttgt ccaacaacgg ttacgacgtg gtgaacttgg gcatcaagca gccactgtcc 2400
   gccatgttgg aagcagcgga agaacacaaa gcagacgtca tcggcatgtc gggacttctt 2460
   gtgaagtcca ccgtggtgat gaaggaaaac cttgaggaga tgaacaacgc cggcgcatcc 2520
   aattacccag tcattttggg tggcgctgcg ctgacgcgta cctacgtgga aaacgatctc 2580
   aacgaggtgt acaccggtga ggtgtactac gcccgtgatg ctttcgaggg cctgcgcctg 2640
   atggatgagg tgatggcaga aaagcgtggt gaaggacttg atcccaactc accagaagct 2700
   attgagcagg cgaagaagaa ggcggaacgt aaggctcgta atgagcgttc ccgcaagatt 2760
   gccgcggagc gtaaagctaa tgcggctccc gtgattgttc cggagcgttc tgatgtctcc 2820
   accgatactc caaccgcggc accaccgttc tggggaaccc gcattgtcaa gggtctgccc 2880
   ttggcggagt tcttgggcaa ccttgatgag cgcgccttgt tcatggggca gtggggtctg 2940
   aaatccaccc gcggcaacga gggtccaagc tatgaggatt tggtggaaac tgaaggccga 3000
   ccacgcctgc gctactggct ggatcgcctg aagtctgagg gcattttgga ccacgtggcc 3060
   ttggtgtatg gctacttccc agcggtcgcg gaaggcgatg acgtggtgat cttggaatcc 3120
   ccggatccac acgcagccga acgcatgcgc tttagcttcc cacgccagca gcgcggcagg 3180
   ttcttgtgca tcgcggattt cattcgccca cgcgagcaag ctgtcaagga cggccaagtg 3240
   gacgtcatgc cattccagct ggtcaccatg ggtaatccta ttgctgattt cgccaacgag 3300
   ttgttcgcag ccaatgaata ccgcgagtac ttggaagttc acggcatcgg cgtgcagctc 3360
   accgaagcat tggccgagta ctggcactcc cgagtgcgca gcgaactcaa gctgaacgac 3420
   ggtggatctg tcgctgattt tgatccagaa gacaagacca agttcttcga cctggattac 3480
   cgcggcgccc gcttctcctt tggttacggt tcttgccctg atctggaaga ccgcgcaaag 3540
   ctggtggaat tgctcgagcc aggccgtatc ggcgtggagt tgtccgagga actccagctg 3600
   cacccagagc agtccacaga cgcgtttgtg ctctaccacc cagaggcaaa gtactttaac 3660
   gtctaa 3666
<210> 11
   <211> 3666
   <212> DNA
   <213> Artificial
<220>
   <223> optimized Corynebacterium glutamicum metH
<400> 11
   atgtcgactt cagttacttc accagcccac aacaacgcac attcctccga atttttggat      60
   gcgttggcaa accatgtgtt gatcggcgac ggcgccatgg gcacccagct ccaaggcttt     120
   gacctggacg tggaaaagga tttccttgat ctggagggct gtaatgagat tctcaacgac     180
   acccgccctg atgtgttgag gcagattcac cgcgcctact ttgaggcggg agctgacttg     240
   gttgagacca atacttttgg ttgcaacctg ccgaacttgg cggattatga catcgctgat     300
   cgttgccgtg agcttgccta caagggcact gcagtggcta gggaagtggc tgatgagatg     360
   ggcccgggcc gaaacggcat gcggcgtttc gtggttggtt ccctgggacc tggaacgaag     420
   cttccatcgc tgggccatgc accgtatgca gatttgcgtg gccactacaa ggaagcagcg     480
   cttggcatca tcgacggtgg tggcgatgcc tttttgattg agactgctca ggacttgctt     540
   caggtcaagg ctgcggttca cggcgttcaa gatgccatgg ctgaacttga tacattcttg     600
   cccattattt gccacgtcac cgtagagacc accggcacca tgctcatggg ttctgagatc     660
   ggtgccgcgt tgacagcgct gcagccactg ggtatcgaca tgattggtct gaactgcgcc     720
   accggcccag atgagatgag cgagcacctg cgttacctgt ccaagcacgc cgatattcct     780
   gtgtcggtga tgcctaacgc aggtcttcct gtcctgggta aaaacggtgc agaataccca     840
   cttgaggctg aggatttggc gcaggcgctg gctggattcg tctccgaata tggcctgtcc     900
   atggtgggtg gttgttgtgg caccacacct gagcacatcc gtgcggtccg cgatgcggtg     960
   gttggtgttc cagagcagga aacctccaca ctgaccaaga tccctgcagg ccctgttgag    1020
   caggcctccc gcgaggtgga gaaagaggac tccgtcgcgt cgctgtacac ctcggtgcca    1080
   ttgtcccagg aaaccggcat ttccatgatc ggtgagcgca ccaactccaa cggttccaag    1140
   gcattccgtg aggcaatgct gtctggcgat tgggaaaagt gtgtggatat tgccaagcag    1200
   caaacccgcg atggtgcaca catgctggat ctttgtgtgg attacgtggg acgagacggc    1260
   accgccgata tggcgacctt ggcagcactt cttgctacca gctccacttt gccaatcatg    1320
   attgactcca ccgagccaga ggttattcgc acaggccttg agcacttggg tggacgaagc    1380
   atcgttaact ccgtcaactt tgaagacggc gatggccctg agtcccgcta ccagcgcatc 1440
   atgaaactgg taaagcagca cggtgcggcc gtggttgcgc tgaccattga tgaggaaggc 1500
   caggcacgta ccgctgagca caaggtgcgc attgctaaac gactgattga cgatatcacc 1560
   ggcagctacg gcctggatat caaagacatc gttgtggact gcctgacctt cccgatctct 1620
   actggccagg aagaaaccag gcgagatggc attgaaacca tcgaagccat ccgcgagctg 1680
   aagaagctct acccagaaat ccacaccacc ctgggtctgt ccaatatttc cttcggcctg 1740
   aaccctgctg cacgccaggt tcttaactct gtgttcctca atgagtgcat tgaggctggt 1800
   ctggactctg cgattgcgca cagctccaag attttgccga tgaaccgcat tgatgatcgc 1860
   cagcgcgaag tggcgttgga tatggtctat gatcgccgca ccgaggatta cgatccgctg 1920
   caggaattca tgcagctgtt tgagggcgtt tctgctgccg atgccaagga tgctcgcgct 1980
   gaacagctgg ccgctatgcc tttgtttgag cgtttggcac agcgcatcat cgacggcgat 2040
   aagaatggcc ttgaggatga tctggaagca ggcatgaagg agaagtctcc tattgcgatc 2100
   atcaacgagg accttctcaa cggcatgaag accgtgggtg agctgtttgg ttccggacag 2160
   atgcagctgc cattcgtgct gcaatcggca gaaaccatga aaactgcggt ggcctatttg 2220
   gaaccgttca tggaagagga agcagaagct accggatctg cgcaggcaga gggcaagggc 2280
   aaaatcgtcg tggccaccgt caagggtgac gtgcacgata tcggcaagaa cttggtggac 2340
   atcattttgt ccaacaacgg ttacgacgtg gtgaacttgg gcatcaagca gccactgtcc 2400
   gccatgttgg aagcagcgga agaacacaaa gcagacgtca tcggcatgtc gggacttctt 2460
   gtgaagtcca ccgtggtgat gaaggaaaac cttgaggaga tgaacaacgc cggcgcatcc 2520
   aattacccag tcattttggg tggcgctgcg ctgacgcgta cctacgtgga aaacgatctc 2580
   aacgaggtgt acaccggtga ggtgtactac gcccgtgatg ctttcgaggg cctgcgcctg 2640
   atggatgagg tgatggcaga aaagcgtggt gaaggacttg atcccaactc accagaagct 2700
   attgagcagg cgaagaagaa ggcggaacgt aaggctcgta atgagcgttc ccgcaagatt 2760
   gccgcggagc gtaaagctaa tgcggctccc gtgattgttc cggagcgttc tgatgtctcc 2820
   accgatactc caaccgcggc accaccgttc tggggaaccc gcattgtcaa gggtctgccc 2880
   ttggcggagt tcttgggcaa ccttgatgag cgcgccttgt tcatggggca gtggggtctg 2940
   aaatccaccc gcggcaacga gggtccaagc tatgaggatt tggtggaaac tgaaggccga 3000
   ccacgcctgc gctactggct ggatcgcctg aagtctgagg gcattttgga ccacgtggcc 3060
   ttggtgtatg gctacttccc agcggtcgcg gaaggcgatg acgtggtgat cttggaatcc 3120
   ccggatccac acgcagccga acgcatgcgc tttagcttcc cacgccagca gcgcggcagg 3180
   ttcttgtgca tcgcggattt cattcgccca cgcgagcaag ctgtcaagga cggccaagtg 3240
   gacgtcatgc cattccagct ggtcaccatg ggtaatccta ttgctgattt cgccaacgag 3300
   ttgttcgcag ccaatgaata ccgcgagtac ttggaagttc acggcatcgg cgtgcagctc 3360
   accgaagcat tggccgagta ctggcactcc cgagtgcgca gcgaactcaa gctgaacgac 3420
   ggtggatctg tcgctgattt tgatccagaa gacaagacca agttcttcga cctggattac 3480
   cgcggcgccc gcttctcctt tggttacggt tcttgccctg atctggaaga ccgcgcaaag 3540
   ctggtggaat tgctcgagcc aggccgtatc ggcgtggagt tgtccgagga actccagctg 3600
   cacccagagc agtccacaga cgcgtttgtg ctctaccacc cagaggcaaa gtactttaac 3660
   gtctaa 3666
<210> 12
   <211> 31
   <212> DNA
   <213> Artificial
<220>
   <223> Primer old 540
<400> 12
   actatgacgt cggcgttgaa gtcctgattg g 31
<210> 13
   <211> 20
   <212> DNA
   <213> Artificial
<220>
   <223> Primer old 541
<400> 13
   tgttacatct tctccggtgc 20
<210> 14
   <211> 43
   <212> DNA
   <213> Artificial
<220>
   <223> Primer old 542
<400> 14
   gcaccggaga agatgtaaca atggctaccg ttgaaaactt caa 43
<210> 15
   <211> 39
   <212> DNA
   <213> Artificial
<220>
   <223> Primer Old 543
<400> 15
   ggtcaggcgt cgaaaagcgt tatgcttcca gggacagga 39
<210> 16
   <211> 19
   <212> DNA
   <213> Artificial
<220>
   <223> Primer old 544
<400> 16
   cgcttttcga cgcctgacc 19
<210> 17
   <211> 34
   <212> DNA
   <213> Artificial
<220>
   <223> Primer Old 545
<400> 17
   actacttcta gacgacaact cctactacct ctcc 34
<210> 18
   <211> 19
   <212> DNA
   <213> Artificial
<220>
   <223> Primer old 494
<400> 18
   aaccgtggaa aacttcaac 19
<210> 19
   <211> 19
   <212> DNA
   <213> Artificial
<220>
   <223> Primer Old 499
<400> 19
   ttccagggac aggatatca 19

## Description

### OBJECT OF THE INVENTION

The present invention relates to a method of increasing the amount of at least one polypeptide in a host cell wherein the codon usage of the nucleotide sequence which is to be expressed is adjusted to the codon usage of abundant proteins of the host cell.

The present invention also relates to nucleotide sequences encoding for a polypeptide with a codon usage that has been adjusted to the codon usage of abundant proteins in the host cell. Such nucleotide sequences allow for increased expression of the respective polypeptide.

The present disclosure is also concerned with a method of increasing the amount of at least one polypeptide in *Corynebacterium glutamicum* wherein the codon usage of the nucleotide sequence which is to be expressed is adjusted to the codon usage of *Corynebacteriun glutamicum.*

The present disclosure also relates to nucleotide sequences encoding for a polypeptide with a codon usage that has been adjusted to the codon usage of *Corynebacterium glutamicum.* Such nucleotide sequences allow for increased expression of the respective polypeptide.

The present disclosure further relates to the use of the aforementioned nucleotide sequences for overexpressing the polypeptides encoded thereby to increase production of fine chemicals.

### BACKGROUND

In a lot of biotechnological processes it is necessary to modulate gene expression. Thus, for some applications it is necessary to increase the expression of a certain gene product and to thereby increase the amount and/or activity of e.g. a protein in the host cell in which the gene of interest is (over)expressed. Similarly, it may be desirable to reduce the amount of expression of an endogenous gene in a host cell.

The fermentative production of so-called fine chemicals is today typically carried out in microorganisms such as *Corynebacterium glutamicum* (*C*. *glutamicum), Escherichia coli (E.coli), Saccharomyces cerevisiae* (*S. cerevisiae*), *Schizzosaccharomycs pombe* (*S. pombe*), *Pichia pastoris* (*P. pastoris*), *Aspergillus niger, Bacillus subtilis, Ashbya gossypii* or *Gluconobacter oxydans.*

Fine chemicals which include e.g. organic acids such as lactic acid, proteogenic or non-proteogenic amino acids, purine and pyrimidine bases, carbohydrates, aromatic compounds, vitamins and cofactors, lipids, saturated and unsaturated fatty acids are typically used and needed in the pharmaceutical, agriculture, cosmetic as well as food and feed industry.

As regards for example the amino acid methionine, currently worldwide annual production amounts to about 500,000 tons. The current industrial production process is not by fermentation but a multi-step chemical process. Methionine is the first limiting amino acid in livestock of poultry feed and due to this mainly applied as a feed supplement. Various attempts have been published in the prior art to produce methionine e.g. using microorganisms such as *E. coli.*

Other amino acids such as glutamate, lysine, threonine and threonine, are produced by e.g. fermentation methods. For these purposes, certain microorganisms such as *C*. *glutamicum* have proven to be particularly suited. The production of amino acids by fermentation has the particular advantage that only L-amino acids are produced and that environmentally problematic chemicals such as solvents as they are typically used in chemical synthesis are avoided.

A lot of the attempts in the prior art to produce fine chemicals such as amino acids, lipids, vitamins or carbohydrates in microorganisms such as *E. coli* and *C. glutamicum* have attempted to achieve this goal by e.g. increasing the expression of genes involved in the biosynthetic pathways of the respective fine chemicals. If e.g. a certain step in the biosynthetic pathway of an amino acid such as methionine or lysine is known to be rate-limiting, over-expression of the respective enzyme may allow obtaining a microorganism that yields more product of the catalysed reaction and therefore will ultimately lead to an enhanced production of the respective amino acid.

WO 2006/008908 describes nucleic sequences for regulating gene transcription and expression.

Attempts to increase production of e.g. methionine and lysine by upregulating the expression of genes being involved in the biosynthetic pathway of methionine or lysine production are e.g. described in WO 02/10209, WO 2006008097, WO2005059093 or in Cremer et al. (Appl. Environ. Microbiol, (1991), 57(6), 1746-1752).

Typically, overexpression of a certain gene in a microorganism such as *E. coli* or *C*. *glutamicum* or other host cells such as *P. pastoris, A. niger* or even mammalian cell culture systems may be achieved by transforming the respective cell with a vector that comprises a nucleotide sequence encoding for the desired protein and which further comprises elements that allow the vector to drive expression of the nucleotide sequence encoding e.g. for a certain enzyme. Using this approach foreign proteins, i.e. proteins that are encoded by sequences that are not naturally found in the host cell that is used for expression, as well as endogenous host cell-specific proteins may be overexpressed. Other typical methods include increasing the copy number of the respective genes in the chromosome, inserting strong promoters for regulating the transcription of the chromosomal copy of the respective genes and enhancing translational initiation by optimization of the ribosomal binding site (RBS).

The expression of foreign genes in a certain host cell may be particularly desirable as this approach allows to confer novel and unique characteristics to a host cell if e.g. a gene encoding for a certain enzymatic activity is introduced which naturally is not found in the host cell.

However, overexpression of foreign genes having no counterpart in the host cell by using e.g. expression vectors such as plasmids has encountered problems. The same has been observed for overexpression of genes which have a counterpart in the host organism as regards their function but which use a nucleotide sequence that is typically not found within the host organism. The failure of host cells such as *E. coli* or *C*. *glutamicum* to express certain foreign (heterologous) sequences may be due to altered codon usage (see e.g. WO 2004042059).

The genetic code is degenerate which means that a certain amino acid may be encoded by a number of different base triplets. Codon usage refers to the observation that a certain organism will typically not use every possible codon for a certain amino acid with the same frequency. Instead an organism will typically show certain preferences, i.e. a bias for specific codons meaning that these codons are found more frequently in the transcribed genes of an organism. One explanation for different codon usages in different organisms may be that the genes encoding for the respective tRNA and tRNA isoacceptors differ in the degree to which they are expressed and thus available during translation.

Eikmanns (1992) J. Bacteriol. 174 (19):6076-6086 discloses the codon usage in the *C*. *glutamicum* genes *gap, pgk* and *tpi.*

Organism-specific codon usage can be one of the reasons why e.g. translation of a synthetic gene or a foreign gene even when coupled to a strong promoter often proceeds much more slowly than would be accepted. This lower than expected translation efficiency is explained by that the protein's coding regions of the gene have a codon usage pattern that does not resemble that of the host cells.

As codon usage is highly biased and varies considerably in different organisms, introduction of a foreign sequence that has a different codon usage bias than the host organism can alter a peptide elongation rates as the host organism will have to produce e.g. more of the respective tRNAs.

There are different codon-optimisation techniques available for improving the translational kinetics of translationally inefficient protein coding regions. These techniques mainly rely on identifying the codon usage for a certain host organism. If a certain gene or sequence should be expressed in this organism, the coding sequence of such genes and sequences will then be modified such that one will replace codons of the sequence of interest by more frequently used codons of the host organism.

However, even for known codon optimisation approaches, there remain efficiency problems as regards the expression of e.g. foreign genes.

In view of this situation, it is one object of the present invention to provide codon usage data for industrially important microorganisms such as *C*. *glutamicum* on the basis of which improved expression of coding sequences can be achieved. Furthermore, it is an object of the present invention to provide new methods for codon optimisation which allow circumvention of the drawbacks of the prior art.

These and other objectives as they will become apparent from the ensuing description of the invention are solved by the present invention as described in the independent claims. The dependent claims relate to preferred embodiments.

### SUMMARY OF THE INVENTION

In one aspect the invention is concerned with a method of increasing the amount of at least one polypeptide in a host cell. The method comprises the step of expressing a polypeptide-encoding sequence which has been adjusted to the codon usage of abundant proteins of the host organism.

In particular, the method comprises the step of expressing a modified nucleotide sequence which encodes for said at least one polypeptide in said host cell. The modified nucleotide sequence is derived from a different starting nucleotide sequence such that the codon usage of the modified nucleotide sequence is adjusted to the codon usage of abundant proteins of the respective host organism. The modified nucleotide sequence and the starting nucleotide sequence encode for the same amino acid sequence. Both sequences encode for identical amino acid sequences.

Modification of the starting nucleotide sequence will usually be done by replacing at least one codon of the starting nucleotide sequence by a codon that is more frequently used in the group of abundant polypeptides of the host organism.

As regards increasing the expression of polypeptides on the basis of the codon usage of abundant host proteins, at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all codons of the starting nucleotide sequence are replaced in the modified nucleotide sequence by more frequently and preferably by the most frequently used codons for the respective amino acid as determined for the group of abundant proteins. In a particularly preferred embodiment the afore-mentioned number of codons to be replaced refers to rare, very rare and particularly extremely rare codons. In an even more preferred embodiment these codons are replaced by frequent, very frequent, extremely frequent or the most frequent codons. In another particularly preferred embodiment, the number of codons to be replaced refers to the least frequently used codons which are replaced by the most frequently used codons.

In one embodiment of the invention, the method will make use of modified nucleotide sequences which use for each amino acid the most frequently used codon of the abundant proteins of the respective host cell.

The at least one polypeptide that is expressed according to the above described method is a polypeptide of said host cell, i.e. an endogenous polypeptide with the proviso that the modified nucleotide sequence is different from the starting sequence encoding a polypeptide of the same amino acid.

Using the inventive method, it is possible to overexpress a polypeptide in a host cell. Thus, using the inventive method the amount of the expressed polypeptide may be increased by at least 5%, 10%, 20%, 30%, 40%, 50%, 60%, 70%, 80%, 90% or 100%. In preferred embodiments the amount of the polypeptide may be increased by a factor of a least 3, 4, 5, 6, 7, 8, 9 or 10 or even more preferably by a factor of at least 20, 50, 100, 500 or 1,000. The increased amount of expressed polypeptide refers to a comparison of expression of the modified nucleotide sequence with expression of the starting nucleotide sequence under comparable conditions (e.g. same host cell, same vector type etc.).

In a preferred embodiment, a method in accordance with the invention relates to increasing the amount of at least one polypeptide in the genus Corynebacterium. A particularly preferred embodiment relates to increasing the amount in *C*. *glutamicum.*

The method of the invention comprises the step of expressing a modified nucleotide sequence encoding for an endogenous polypeptide. The modified nucleotide sequence is derived from a different starting nucleotide sequence such that the codon usage of the modified nucleotide sequence is adjusted to the codon usage of the group of abundant proteins of *C*. *glutamicum.* Both the modified and the starting nucleotide sequence will encode for the same amino acid sequence. Both sequences encode for identical amino acid sequences

As set out above, this embodiment of the invention may be used to overexpress endogenous polypeptides.

As regards the expression of polypeptides in the species of *C*. *glutamicum* by modified codon usage, at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all codons of the starting nucleotide sequences are replaced in the modified nucleotide sequence by more frequently and preferably by the most frequently used codons for the respective amino acid as determined for the group of abundant proteins. In a particularly preferred embodiment, the afore-mentioned number of codons to be replaced refers to rare, very rare and particularly extremely rare codons. In an even more preferred embodiment these codons are replaced by frequent, very frequent, extremely frequent or the most frequent codons. In another particularly preferred embodiment, the number of codons to be replaced refers to the least frequently used codons which are replaced by the most frequently used codons.

For expression of polypeptides in the species of *C*. *glutamicum* by optimised codon usage, in another embodiment the starting nucleotide sequence encoding for the polypeptide may be modified such that at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all codons of the starting nucleotide sequence are replaced in the resulting modified nucleotide sequence by the most frequently used codons for the respective amino acid according to Table 2. In particularly preferred embodiment the afore-mentioned number of codons to be replaced refers to rare, very rare and particularly extremely rare codons. In another particularly preferred embodiment, the number of codons to be replaced refers to the least frequently used codons which are replaced by the most frequently used codons of Table 2.

Further preferred embodiments of the invention relate to methods of increasing the amount of a polypeptide in *C. glutamicum* wherein at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all codons of the starting nucleotide sequence are replaced in the resulting modified nucleotide sequence by the most frequently used codon for the respective amino acid according to Table 2. In an even more preferred embodiment the afore-mentioned number of codons to be replaced refers to rare, very rare and particularly extremely rare or the least frequently used codons.

In a particularly preferred embodiment of the invention, the method will rely on modified nucleotide sequences using the codons GUU for valine, GCU for alanine, GAC for aspartic acid, GAG for glutamic acid and/or ATG for methionine if ATG is the start codon.

In yet another embodiment of the invention which relates to a method of increasing the amount of polypeptide in *C*. *glutamicum,* at least one codon of the aforementioned modified nucleotide sequences will be selected from Table 3.

Another particularly preferred embodiment of the invention relates to methods of increasing the amount of a polypeptide in Corynebacterium and particularly preferably in *C*. *glutamicum* wherein at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all codons of the starting nucleotide sequence are replaced in the resulting modified nucleotide sequence by the codons for the respective amino acid according to Table 3. In an even more preferred embodiment the afore-mentioned number of codons to be replaced refers to rare, very rare and particularly extremely rare codons.

Another particularly preferred embodiment of the invention relate to methods of increasing the amount of a polypeptide in *C*. *glutamicum* wherein all codons of the starting nucleotide sequence are replaced in the resulting modified nucleotide sequence by the codons for the respective amino acid according to Table 3.

As regards the embodiments of the invention that relate to increasing the amount of a polypeptide in the host organism *C*. *glutamicum* by using codon optimisation that is based on the codon usage of abundant proteins in *C*. *glutamicum,* the methods may be used to overexpress the at least one polypeptide by the same amounts as has been set out above in general. Again, the increase in amount of polypeptide obtained by expression following a method in accordance with the invention is determined in comparison to expression of the starting original sequence in *C*. *glutamicum.*

In some of the preferred embodiments of the invention, the above described method of increasing the amount of a polypeptide in *C*. *glutamicum* may be used for producing fine chemicals such as amino acids, sugars, lipids, oils, carbohydrates, vitamins, cofactors etc.

For these purposes the modified nucleotide sequences may be selected from sequences encoding genes of biosynthetic pathways that are involved in the production of the aforementioned fine chemicals and for which overexpression is known to enhance production of the fine chemical(s).

In one particularly preferred embodiment, methods in accordance with the invention may thus be used to produce fine chemicals such as amino acids and particularly amino acids such as lysine, threonine, cysteine and methionine.

Yet another embodiment of the present invention relates to the modified nucleotide sequences which are used for expression of a polypeptide in a host cell that have been derived from the different starting nucleotide sequences encoding for polypeptides of the same amino acid sequence by adjusting the codon usage of the modified nucleotide sequences to the codon usage of the group of abundant proteins of the respective host cell.

Of course, the invention in a preferred embodiment also relates to such modified nucleotide sequences that have been derived for a specific polypeptide of endogenous origin without additional mutations, by replacing at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all codons of the starting nucleotide sequences in the modified nucleotide sequence by the most frequently used codons for the respective amino acid as determined for the group of abundant proteins of the respective host organism. In an even more preferred embodiment the afore-mentioned numbers of codons to be replaced refer to rare, very rare and particularly extremely rare codons.

In the invention, the modified nucleotide sequence uses for each amino acid the most frequently used codon of the abundant proteins of the respective host cell.

In case of modified nucleotide sequences that are to be (over)expressed in *C. glutamicum,* at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the starting nucleotide sequences are replaced in the modified nucleotide sequence by the most frequently used codons for the respective amino acid as determined for the group of abundant proteins of *C*. *glutamicum.* In an even more preferred embodiment the afore-mentioned numbers of codons to be replaced refer to rare, very rare and particularly extremely rare codons.

For other preferred embodiments as far as nucleotide sequences for increasing expression in *C. glutamicum* is concerned, at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all codons of the starting nucleotide sequence are replaced in the resulting modified nucleotide sequence by the most frequently used codon for the respective amino acid according to Table 2. In an even more preferred embodiment the afore-mentioned number of codons to be replaced refers to rare, very rare and particularly extremely rare codons.

*A*s regards a particularly preferred embodiment of the modified nucleotide sequences, codons of the modified nucleotide sequence will use GUU for valine, GCU for alanine, GAC for aspartic acid, GAG for glutamic acid and/or ATG for methionine if ATG is the start codon.

The modified nucleotide sequence which is used for expression of the polypeptide in *C*. *glutamicum* uses codons that are selected from the codon usage of Table 3.

Thus, another particularly preferred embodiment of the invention relates to recombinant modified nucleotide sequences for increasing the amount of a polypeptide in *C. glutamicum* wherein at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all codons of the starting nucleotide sequence are replaced in the resulting modified nucleotide sequence by the codons for the respective amino acid according to Table 3. In an even more preferred embodiment the afore-mentioned numbers of codons to be replaced refer to rare, very rare and particularly extremely rare codons.

Another particularly preferred embodiment of the invention relates to modified nucleotide sequences for increasing the amount of a polypeptide in *C*. *glutamicum* wherein all codons of the starting nucleotide sequence are replaced in the resulting modified nucleotide sequence by the codons for the respective amino acid according to Table 3.

Other embodiments of the disclosure relate to vectors that comprise the aforementioned nucleotide sequences and which are suitable for expression of a polypeptide in a host cell.

Yet another embodiment of the present disclosure relates to host cells which comprise the aforementioned modified nucleotide sequences or the aforementioned vectors.

The present invention also relates to the use of the aforementioned methods, nucleotide sequences, vectors and/or host cells for producing fine chemicals such as amino acids, lipids, oils, carbohydrates, vitamins, cofactors etc.

The aforementioned methods, nucleotide sequences, vectors and host cells may particularly be used for production of fine chemicals such as amino acids including lysine, threonine, cysteine, and methionine.

The above described methods for increasing the amount of a polypeptide in a host cell rely on an optimisation of codon usage on the basis of the codon frequency of abundant proteins of the respective host cell. Optimisation means that when designing the modified nucleotide sequence preferably such codons are avoided which have been found to be rarely used in the group of abundant proteins of the respective host cells. Instead such codons are selected that are more (and preferably most) frequently used for the specific amino acid according to the codon usage of abundant proteins of the host cell.

However, the present disclosure not only relates to codon optimisation as described above, but in one embodiment also to preserving the distribution frequency of codon usage in the original starting sequence and the modified sequence. For example, instead of replacing a rarely used codon in the original starting sequence with a more frequently used host-specific codon, one may substitute the codon of the starting sequence with a codon of the host cell that is used at a comparable frequency in abundant proteins of the host cell. As far as *C*. *glutamicum* in particular is concerned, one may rely in this context also on the data of Table 2 from which one can infer the distribution frequency of codons in abundant proteins.

Of course, the present disclosure also relates to nucleotide sequences in which the distribution frequency of codon usage is adjusted to the distribution frequency of codon usage of abundant proteins. Similarly, vectors and host cells comprising such nucleotide sequences form part of the disclosure as well as the use of such methods, nucleotide sequences, vectors and host cells for producing fine chemicals.

Yet another embodiment of the present disclosure relates to methods for increasing the amount of polypeptide in *C. glutamicum* in which a modified nucleotide sequence is expressed wherein the sequence of the modified nucleotide sequence has been adjusted to the codon usage of the complete organism *C*. *glutamicum* as set forth in Table 1. In methods relating to this aspect of the disclosure a modified nucleotide sequence may be used wherein at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the rare codons of the starting nucleotide sequence are replaced in the resulting modified nucleotide sequence by more frequently and preferably the most frequently used codons for the respective amino acid according to Table 1. In an even more preferred embodiment the afore-mentioned numbers of codons to be replaced refer to rare, very rare and particularly extremely rare codons.

In another preferred disclosure of this latter aspect of the invention, a modified nucleotide sequence may be used wherein all codons of the starting nucleotide sequence are replaced in the resulting modified nucleotide sequence by more frequently and preferably the most frequently used codons for the respective amino acid according to Table 1.

The present disclosure also relates to the nucleotide sequences which have been optimised on the basis of the codon usage of the organism *C. glutamicum.* Cloning and expression vectors and host cells which comprise these sequences also form part of the disclosure. The present disclosure relates as well to the use of such sequences for producing fine chemicals such as those mentioned above.

### FIGURES

Figure 1 a) shows the codon usage optimised sequence of Lysine-2,3-aminomutase of *Clostridium subterminale* (SEQ ID No. 1). Figure 1 b) shows the complete insert which has been cloned into pClik 5a MCS (p Clik 5a MCS Psod Synth Kam A, SEQ ID No 2.). Underlined are the SpeI-recognition sites. The pSOD promoter is in italics. The codon usage optimised sequence of Lysine-2,3-aminomutase is in bold and the terminator sequence is grey shadowed.
Figure 2 shows the expression constructs that were used for expressing the non-modified sequence and codon usage optimised Lysine-2,3-aminomutase of *C*. *subterminale.*
Figure 3 shows an SDS-PAGE gel picture in which expression of codon usage optimised versus non-modified Lysine-2,3-aminomutase expression of *C*. *subterminale* was determined in *C*. *glutamicum.* Lanes M represent a pre-stained protein standard (SeeBlue Prestained Standard, Invitrogen). Lanes 1,2 represent expression from pClik 5a MCS. Lanes 3,4 represent expression from pClik 5a MCS Psod synth. KamA (Fig. 2a). Lanes 5,6 represent expression from pClik 5a MCS genomisch KamA Cl sub (Fig. 2b).
Figure 4 shows the expression construct that was used for expressing the non-modified sequence of Lysine-2,3-aminomutase of *C*. *subterminale* under the control of the Psod promoter.
Figure 5 shows an SDS-PAGE gel picture in which expression of codon usage optimised versus non-modified lysA was determined in *C*. *glutamicum.* Lane 1 represents a pre-stained protein standard (SeeBlue Prestained Standard, Invitrogen). Lanes 2,3 represent expression from pClik 5a MCS. Lanes 4,5 represent expression from pClik 5a MCS genomisch KamA Cl sub (Fig. 2b). Lanes 6,7 represent expression from pClik 5a MCS Psod synth. KamA (Fig. 2a). Lanes 8,9 represent expression from pClik 5a MCS genom KamA (Fig. 4). The arrow indicates lysA.
Figure 6 shows the codon usage optimised sequence of diaminopimelate decarboxylase (lysA (SEQ ID No. 8).
Figure 7 shows the codon usage optimised sequence of lysA including up- and downstream regions. The restriction sites are underlined and the coding sequence is in bold. The upstream and downstream sequences are in italics (SEQ ID No. 9).
Figure 8 shows the expression construct of codon usage optimised lysA for expression in *C. glutamicum.*

Figure 9 shows an SDS-PAGE gel picture in which expression of codon usage optimised versus non-modified metH was determined in *C*. *glutamicum.* Lanes 1-3 represent expression of optimised metH. Lane 4 represents expression from empty vector. Lane 5 represents expression of wild type metH. The arrow indicates metH.

### DETAILED DESCRIPTION OF THE INVENTION

In one aspect, the present invention relies partly on the surprising finding that determination of the codon usage of an organism may give different results depending on whether the codon usage is determined only for abundant proteins or for the organism as a whole.

Typically, codon usage tables in the prior art for organisms such as *E.coli* etc. have been based on an analysis of the complete genome. The inventors of the present invention have surprisingly found for the case of *C*. *glutamicum* that codon usage analysis of abundant proteins will give quite different results compared to codon usage frequencies as determined for the complete organism of *C*. *glutamicum.* Without being wanted to be bound to a theory, it is assumed that the specific codon usage frequency of abundant proteins in an organism such as *C*. *glutamicum* reflects certain requirements as to the codon composition of a highly expressed nucleotide sequence.

The specific codon usage distribution of highly expressed genes may e.g. reflect preferences for codons that are recognised by tRNAs that are also frequently and abundantly available in the host organisms' cells. Similarly such codons may reflect transcript RNA structures that for their spatial arrangement can be more efficiently translated.

Codons that are frequently used in abundant proteins may have been selected for their ability to drive expression. Similarly, codons which are only rarely used in abundant proteins may be prime targets for replacement by other more frequently used codons.

Identifying codon usage frequencies not on the basis of the whole organism, but for abundant proteins thus opens the intriguing possibility of defining specific optimized codon usage information that may be used for overexpression of foreign genes, endogenous genes or mutated versions thereof in a host organism.

It seems reasonable to assume that the finding that highly expressed proteins in a host cell have a different codon usage compared to the situation where codon usage for all genes of an organism is determined will not be limited to *C*. *glutamicum* but also be observed for other organisms such as *E. coli,* yeast cells, plant cells, insect cells or mammalian cell culture cells. In view of these surprising findings, the present invention relates to a method of increasing the amount of at least one endogenous polypeptide in *C. glutamicum,* comprising the step of expressing in *C*. *glutamicum* a modified nucleotide sequence encoding for said at least one polypeptide, wherein the codon usage of the modified nucleotide sequence is adjusted to the codon usage of abundant proteins of *C*. *glutamicum,* wherein said modified nucleotide sequence is derived from a different starting nucleotide sequence and wherein the modified and starting nucleotide sequence encode for the same amino acid sequence and wherein at least one, some or all codons of said modified nucleotide sequence are selected from the codon usage of Table 3.

In the context of the present invention, the term "increasing the amount of at least one polypeptide in a host cell" refers to the situation that upon expressing the modified nucleotide sequences in the host cell, a higher amount of this polypeptide is produced in a host cell compared to the situation where a non-modified starting nucleotide sequence encoding for a polypeptide of the same amino acid sequence is expressed in the same type of host cells under similar conditions such as e.g. comparable transfection procedures, comparable expression vectors etc.

The term "host cell" or "organism" for the purposes of the present disclosure refers to any organism that is commonly used for expression of nucleotide sequences for production of e.g. polypeptides. In particular the term "host cell" or "organism" relates to prokaryotes, lower eukaryotes, plants, insect cells or mammalian cell culture systems.

The organisms of the present disclosure thus comprise yeasts such as S. *pombe* or S. *cerevisiae* and *Pichia pastoris.*

Plants are also considered by the present disclosure for overexpressing polypeptides. Such plants may be monocots or dicots such as monocotyledonous or dicotyledonous crop plants, food plants or forage plants. Examples for monocotyledonous plants are plants belonging to the genera of avena (oats), triticum (wheat), secale (rye), hordeum (barley), oryza (rice), panicum, pennisetum, setaria, sorghum (millet), zea (maize) and the like.

Dicotyledonous crop plants comprise inter alias cotton, leguminoses like pulse and in particular alfalfa, soybean, rapeseed, tomato, sugar beet, potato, ornamental plants as well as trees. Further crop plants can comprise fruits (in particular apples, pears, cherries, grapes, citrus, pineapple and bananas), oil palms, tea bushes, cacao trees and coffee trees, tobacco, sisal as well as, concerning medicinal plants, rauwolfia and digitalis. Particularly preferred are the grains wheat, rye, oats, barley, rice, maize and millet, sugar beet, rapeseed, soy, tomato, potato and tobacco. Further crop plants can be taken from US 6,137,030.

Mammalian cell culture systems may be selected from the group comprising e.g. NIH T3 cells, CHO cells, COS cells, 293 cells, Jurkat cells and HeLa cells.

The invention relates to the use of host cells which are of the species *Corynebacterium glutamicum*

In other preferred embodiments of the invention the host cells may be selected from the group comprising *Corynebacterium glutamicum* ATCC13032, *Corynebacterium glutamicum* KFCC10065 and *Corynebacterium glutamicum* ATCC21608 as well as strains that are derived thereof by e.g. classical mutagenesis and selection or by directed mutagenesis.

Other particularly preferred strains of C. *glutamicum* may be selected from the group comprising ATCC13058, ATCC13059, ATCC13060, ATCC21492, ATCC21513, ATCC21526, ATCC21543, ATCC13287, ATCC21851, ATCC21253, ATCC21514, ATCC21516, ATCC21299, ATCC21300, ATCC39684, ATCC21488, ATCC21649, ATCC21650, ATCC19223, ATCC13869, ATCC21157, ATCC21158, ATCC21159, ATCC21355, ATCC31808, ATCC21674, ATCC21562, ATCC21563, ATCC21564, ATCC21565, ATCC21566, ATCC21567, ATCC21568, ATCC21569, ATCC21570, ATCC21571, ATCC21572, ATCC21573, ATCC21579, ATCC19049, ATCC19050, ATCC19051, ATCC19052, ATCC19053, ATCC19054, ATCC19055, ATCC19056, ATCC19057, ATCC19058, ATCC19059, ATCC19060, ATCC19185, ATCC13286, ATCC21515, ATCC21527, ATCC21544, ATCC21492, NRRLB8183, NRRL W8182, B12NRRLB12416, NRRLB12417, NRRLB12418 and NRRLB11476.

The abbreviation KFCC stands for Korean Federation of Culture Collection, ATCC stands for American-Type Strain Culture Collection and the abbreviation DSM stands for Deutsche Sammlung von Mikroorganismen. The abbreviation NRRL stands for ARS cultures collection Northern Regional Research Laboratory, Peorea, IL, USA.

Particularly preferred are microorganisms of *Corynebacterium glutamicum* that are already capable of producing fine chemicals such as L-lysine, L-methionine and/or L-threonine. Therefore the strain *Corynebacterium glutamicum* ATCC13032 and derivatives of this strain are particularly preferred.

The term "nucleotide sequence" for the purposes of the present invention relates to any nucleic acid molecule that encodes for polypeptides such as peptides, proteins etc. These nucleic acid molecules may be made of DNA, RNA or analogues thereof. However, nucleic acid molecules being made of DNA are preferred.

The terms "non-modified nucleotide sequence" or "starting nucleotide sequence" for the purposes of the present invention, relates to a nucleotide sequence which is intended to be used for (over) expression in a host cell and which has not been amended with respect to its codon usage in the expression host.

In the present invention wherein an endogenous polypeptide, i.e. a polypeptide that is naturally found within the host cell is to be expressed, the term "non-modified/starting nucleotide sequence" relates to a nucleotide sequence encoding for an endogenous protein which has not been adjusted to the codon usage of abundant proteins of the host cell.

The term "modified nucleotide sequence" for the purposes of the present invention relates to a sequence that has been modified for expression in a host cell by adjusting the sequence of the originally different non-modified/starting nucleotide sequence to the codon usage as used by abundant proteins of the host cell.

The person skilled in the art is clearly aware that modification of the starting nucleotide sequence describes the process of optimization with respect to codon usage.

The term "abundant proteins" for the purposes of the present invention relates to the group of highly expressed genes within a host cell or organism.

The person skilled in the art is familiar with identifying the group of abundant proteins in a host cell or organism. This may be achieved e.g. by 2D gel electrophoresis. In 2D gel electrophoresis, a protein mixture such as a crude cellular extract is separated on protein gels by e.g. size and isoelectric point. Subsequently these gels are stained and the intensity of the various spot is an indication of the overall amount of protein present in the cell.

Using standard software packages one will select a group of proteins whose signal intensities are above a certain threshold background level and will define this group of protein as abundant proteins. Typical software packages used for this purpose include e.g. Melanie3 (Geneva Bioinformatics SA).

The person skilled in the art is well aware that different host cells such as microorganisms, plant cells, insect cells etc. will differ with respect to the number and kind of abundant proteins in a cell. Even within the same organism, different strains may show a somewhat heterogeneous expression profile on the protein level. One will therefore typically analyse different strains and consider such proteins that are found for all strains to be abundant.

A good selection parameter for defining a group of abundant proteins for the purposes of the present invention is to consider only the 10 to 200 and preferably 10 to 30 most abundant proteins as detected in the above described 2D gel electrophoresis procedure. Preferably one will only consider cytosolic proteins for the group of abundant proteins, only.

Thus, in a preferred embodiment the term "abundant proteins" refers to the group of the approximately 13, 14 or 15 abundant proteins in whole cell cytosolic extracts of host organisms as identified by 2D gel electrophoresis.

Once one has identified the abundant proteins, one may use software tools such as the "Cusp" function of the EMBOSS toolbox version 2.2.0 that can be downloaded at HTTP://ENOOSS.sourceforge.net/download/. Other software packages that may be used are available at www.entelechon.com (e.g. Leto 1.0).

In a preferred embodiment of the present invention, optimising a nucleotide sequence for (over) expression in a host cell by codon usage optimisation may be achieved by modifying the above described starting nucleotide sequence encoding for a polypeptide such that the modified nucleotide sequence uses for each amino acid the most frequently used codon as determined for the group of abundant proteins of a host cell. The modified and the starting nucleotide sequence will encode for the same amino acid sequence. Both sequences encode for identical amino acid sequences.

In another preferred embodiment "rare", "very rare", "extremely rare" and preferably the least frequently used codons of the non-modified sequence will be replaced by "frequent" codons in the modified sequence with codon frequency being determined for the group of abundant proteins of the respective host cell.

Unless otherwise indicated the terms "rare" and "frequent" codons refer to the relative frequency by which a certain codon of all possible codons encoding a specific amino acid is used by the group of abundant proteins.

A codon will be considered to be "rare" if it is used less than 20% for the specific amino acid. A "very rare" codon will be used at a frequency of less than 10% and an "extremely rare" codon will be used at a frequency of less than 5%. The frequency is determined on the basis of the codon usage of the abundant proteins of the host organism.

As the amino acids methionine and tryptophane are encoded by one codon only, the respective codon frequency is always 100%. However the amino acid alanine is encoded by four codons, namely GCU, GCC, GCA and GCG. For the whole organism of *C*. *glutamicum* these codons are used at a relative frequency of 23.7 %, 25.4% , 29.3 % and 21.6% (see Table 1, experiment 1). However, in the group of abundant proteins, these codons are used at relative frequencies of 46.8%, 9.9%, 35.9% and 7.4% (see Table 2, experiment 1).

In view of the above explanations the codons GCC and GCG are thus considered to be rare and more precisely to be very rare codons. The replacement of "rare", "very rare" and "extremely rare" codons can prove beneficial because "brakes" of translational efficiency are removed.
Similarly a codon will be considered to be "frequent" if it used at a relative frequency of more than 40%. It is "very frequent" if is used at relative frequency of more than 60% and a relative frequency of more than 80% is indicative of an "extremely frequent" codon. Again the relative frequencies are based on the codon usage of the abundant proteins of the host cell unless otherwise indicated.

The terms "express", "expressing", "expressed" and "expression" refer to expression of a gene product (*e*.*g*., a biosynthetic enzyme of a gene of a pathway) in a host organism. The expression can be done by genetic alteration of the microorganism that is used as a starting organism. In some embodiments, a microorganism can be genetically altered (*e*.*g*., genetically engineered) to express a gene product at an increased level relative to that produced by the starting microorganism or in a comparable microorganism which has not been altered. Genetic alteration includes, but is not limited to, altering or modifying regulatory sequences or sites associated with expression of a particular gene (*e*.*g*. by adding strong promoters, inducible promoters or multiple promoters or by removing regulatory sequences such that expression is constitutive), modifying the chromosomal location of a particular gene, altering nucleic acid sequences adjacent to a particular gene such as a ribosome binding site or transcription terminator, increasing the copy number of a particular gene, modifying proteins (*e*.*g*., regulatory proteins, suppressors, enhancers, transcriptional activators and the like) involved in transcription of a particular gene and/or translation of a particular gene product, or any other conventional means of deregulating expression of a particular gene using routine in the art (including but not limited to use of antisense nucleic acid molecules, for example, to block expression of repressor proteins).

Overexpression for the purposes of the present invention means that the amount of the polypeptide that is to be overexpressed is increased by at least 5%, 10%, 20%, 30%, 40%, 50%, 60%, 70%, 80%, 90% or 100% and preferably by a factor of at least 3, 4, 5, 6, 7, 8, 9 or 10 and more preferably by a factor of at least 20, 50, 100, 500 or 1000 if expression of the modified nucleotide sequence is compared to expression of the starting nucleotide sequence in the same type of host organism under a comparable situation (comparable chromosomal position of the respective sequences, comparable vectors, comparable promoters etc.).

The present invention uses the inventive method for (over) expressing endogenous polypeptides of the host cell, i.e. polypeptides being encoded by sequences that are naturally found within the host cell. For example, a host cell-specific low-abundance protein may be overexpressed by modifying the different starting nucleotide sequence encoding for the low-abundance protein such that the codon usage of the modified nucleotide sequence which in this case may encode for a polypeptide of identical amino acid is adjusted to the codon usage of abundant proteins of the host cell as defined above.

In some embodiments of the disclosure it may be sufficient and preferred to replace rare, very rare and extremely rare codons with more frequently used codons as determined for the group of abundant proteins. In a preferred embodiment the modified nucleotide sequence may use for each of the least frequently used codon the most frequently used codon of the abundant proteins of the host cell.

The above methods may be used for production of fine chemicals as defined below. To this end, the modified nucleotide sequences may be selected from genes which are known to participate in the biosynthesis of such fine chemicals. Particularly preferred are genes for which overexpression is known to stimulate fine chemical production.

A preferred method in accordance with the present invention relates to a method of increasing the amount of at least one polypeptide in *C. glutamicum* comprising the step of expressing a modified nucleotide sequence coding for at least one polypeptide in *C*. *glutamicum* wherein said modified nucleotide sequence is derived from a different starting sequence such that the codon usage of the modified nucleotide sequence is adjusted to the codon usage of abundant proteins of *C. glutamicum.* The modified and starting nucleotide sequence will encode for identical amino acid sequences as described above. The modified and starting nucleotide sequences will thus encode the same amino acid sequence.

Of course, the definitions as provided above for the meaning of the terms "modified nucleotide sequences", "starting (non-modified) nucleotide sequences", "rare codons", "frequent codons" etc. apply equally for these preferred embodiments of the invention.

The abundant proteins of e.g. *C. glutamicum* can be determined as described above by 2D protein gel electrophoresis. To this purpose, *C*. *glutamicum* strains may be cultivated under standard conditions. Then, cell extracts may be prepared using common lysis protocols. After lysis, the cell extracts are centrifuged and approximately 25-50 µg are analyzed by standard 2D-PAGE. An example of the approach can be found below in example 1 as well as in the material and methods part of Hansmeier et al. (Proteomics 2006, 6, 233-250)

Following this approach abundant proteins in *C*. *glutamicum* can be identified by either selecting the most abundant 10 to 300 cytosolic proteins or by identifying 10 to 30 cytosolic proteins that are observed to be present in elevated amounts in various strains. These results are assumed to be representative also for the group of abundant proteins in other Corynebacterium species.

For the purposes of the present invention, the term "abundant proteins of *C*. *glutamicum*" can relate to the group comprising the following protein factors (accession number of nucleotide sequence shown in brackets):
Elongation Factor Tu (Genbank accession no: X77034)
Glycerin-aldehyde-3-phosphate-dehydrogonase (Genbank accession no: BX927152, ±, nt. 289401-288397)
Fructose bisphosphate aldolase (Genbank accession no: BX927156, ±, nt. 134992-133958)
Elongation Factor Ts (Genbank accession no: BX927154, ±, nt. 14902-14075)
Hypothetical protein (Genbank accession no: BX927155, ±, nt. 213489-214325 )
Enolase (Genbank accession no: BX927150, nt. 338561-339838)
Peptidyl-prolyl-Cis-trans isomerase (Genbank accession no: BX927148, nt. 34330-34902)
Superoxide dismutase (Genbank accession no: AB055218)
Phosphoglycerate dehydrogenase (Genbank accession no: BX927151, nt. 306039-307631)
SSU Rib protein SIP (Genbank accession no: BX927152, ±, nt. 26874-28334 )
Triose phosphate-isomerase (Genbank accession no: BX927152, ±, nt. 286884-286105)
Isopropylmalat-synthase (Genbank accession no: X70959 )
Butane-2,3-dioldehydrogenase (Genbank accession no: BX927156, nt. 20798-21574 )
Fumarate-hydratase (Genbank accession no: BX927151, ±, nt. 18803-17394)

On the basis of these aforementioned fourteen proteins, a codon usage table can be created using the aforementioned "CUSP" function of the EMBOSS toolbox.

The above-described group of fourteen proteins may particularly be used for determining or for defining the group of abundant proteins in *C. glutamicum* if the *C. glutamicum* strain ATCC 13032 and/or derivatives (obtained e.g. by classical mutagenesis and selection or genetic engineering) are used in the 2D-gel electrophoresis analysis.

Using the CUSP function of the EMBOSS toolbox version one can thus create a Codon Usage Table that reflects codon usage of abundant proteins of Corynebacterium in general and preferably of *C*. *glutamicum.*

Surprisingly the codon usage of these abundant proteins differs significantly from the codon usage as determined for the whole genome of *C. glutamicum* as becomes clear from a comparison of Tables 1 and 2 (see Experiment 1 below). Codon usage of the whole genome of *C*. *glutamicum* can e.g. be determined from strains that are completely sequenced such as strain ATCC13032 and Codon Usage Tables may e.g. generated by the CUSP function of the aforementioned EMBOSS toolbox or are available at e.g. HTTP://www.kazusa.or.jp. Highly comparable results are obtained if one uses the most abundant cytosolic proteins as mentioned in Table 4 of Hansmeier et al. (*vide supra*).

Similarly a preferred embodiment of the invention relates to the use of modified nucleotide sequences for increasing the amount of a polypeptide in *C*. *glutamicum* wherein the codons GUU for valine, GCU for alanine, GAC for aspartic acid, GAG for glutamic acid and/or ATG for methionine (if it is the start codon) are used.

In a particularly preferred embodiment the method of increasing the amount of a polypeptide in *C*. *glutamicum* comprises the step of expressing a modified nucleotide sequence having been derived from a starting nucleotide sequence wherein the codons of the modified nucleotide sequence are selected for at least one, some and preferably for each amino acid from the codon usage of Table 3.

In all of the aforementioned methods for increasing expression of a polypeptide i in *C*. *glutamicum,* rare, very rare codons and extremely rare codons are preferably exchanged against the most frequently used codons as they can be taken from Table 2.

Of course, the methods that are used to increase the expression of a polypeptide in *C*. *glutamicum* may be used to express endogenous polypeptides of *C*. *glutamicum.*

In a preferred embodiment the above described methods in which the modified nucleotide sequence is e.g. adapted to the codons of Table 3 or the aforementioned codons for valine, alanine, aspartic acid, glutamic acid and/or the start methionine, the host organism is *Corynebacterium glutamicum.*

Also preferred are the above-mentioned *C. glutamicum strain* and particularly preferred is the strain *Corynebacterium glutamicum* ATCC13032 and all its derivatives. The strains ATCC 13286, ATCC 13287, ATCC 21086, ATCC 21127, ATCC 21128, ATCC 21129, ATCC 21253, ATCC 21299, ATCC 21300, ATCC 21474, ATCC 21475, ATCC 21488, ATCC 21492, ATCC 21513, ATCC 21514, ATCC 21515, ATCC 21516, ATCC 21517, ATCC 21518, ATCC 21528, ATCC 21543, ATCC 21544, ATCC 21649, ATCC 21650, ATCC 21792, ATCC 21793, ATCC 21798, ATCC 21799, ATCC 21800, ATCC 21801, ATCC 700239, ATCC 21529, ATCC 21527, ATCC 31269 and ATCC 21526 which are known to produce lysine can also preferably be used. The other aforementioned strains can also be used.

In another embodiment of the invention, a vector that comprises the aforementioned nucleotide sequences is used to drive expression of a modified nucleotide sequence in the host cell, in C. glutamicum for increasing the amount of a polypeptide in these host cells. Such vectors may e.g. be plasmid vectors which are autonomously replicable in coryneform bacteria. Examples are pZ1 (Menkel et al. (1989), Applied and Environmental Microbiology 64: 549-554), pEKEx1 (Eikmanns et al.(1991), Gene 102: 93-98), pHS2-1 (Sonnen et al. (1991), Gene 107: 69-74 ) These vectors are based on the cryptic plasmids pHM1519, pBL1 or pGA1. Other vectors are pCLiK5MCS (WO2005059093), or vectors based on pCG4 (US-A 4,489,160) or pNG2 (Serwold-Davis et al. (1990), FEMS Microbiology Letters 66, 119-124) or pAG1 (US-A 5,158,891).

When optimizing the codon usage, other influencing factors, like the resulting mRNA structure, should also be considered. One should e.g. possibly avoid to generate a mRNA secondary structure which is unstable. Furthermore one should possibly avoid using a codon recognized by the same tRNA in direct physical proximity on the mRNA.

Of course, the above described preferred embodiments of the invention which relate to methods of increasing the amount of a polypeptide in a host cell by using codon usage optimised nucleotide sequences which have been obtained with respect to the codon usage of the above defined group of the approximately fourteen most abundant proteins in *C. glutamicum* can be used to overexpress polypeptides being encoded by the modified nucleotide sequence in *C. glutamicum.*

With overexpression it is meant that the amount of the at least polypeptide is increased by at least 5%, 10%, 20%, 30%, 40%, 50%, 60%, 70%, 80%, 90% or 100% and preferably by a factor of at least 3, 4, 5, 6, 7, 8, 9 or 10 and more preferably by a factor of at least 20, 50, 100, 500 or 1000 if expression of the modified nucleotide sequence is compared to expression of the starting nucleotide sequence under comparable conditions.

It is understood that it is not always desirable to increase expression as much as possible. In certain cases an increase of factor 3 may be sufficient and desirable. The present invention offers the possibility to fine tune repression by e.g. not replacing all codons by the most frequently used codons, but by e.g. exchanging only two or three (rare) codons at selected positions.

A preferred embodiment of the present invention relates to methods of increasing the amount of a polypeptide in *C. glutamicum* wherein the above described modified nucleotide sequences are selected from the group comprising nucleotide sequences encoding genes of biosynthetic pathways of fine chemicals for which overexpression is known to enhance production of the fine chemicals.

The term "fine chemical" is well known to the person skilled in the art and comprises compounds which can be used in different parts of the pharmaceutical industry, agricultural industry as well as in the cosmetics, food and feed industry. Fine chemicals can be the final products or intermediates which are needed for further synthesis steps. Fine chmicals also include monomers for polymer synthesis.

Fine chemicals are defined as all molecules which contain at least two carbon atoms and additionally at least one heteroatom which is not a carbon or hydrogen atom. Preferably fine chemicals relate to molecules that comprise at least two carbon atoms and additionally at least one functional group, such as hydroxy-, amino-, thiol-, carbonyl-, carboxy-, methoxy-, ether-, ester-, amido-, phosphoester-, thioether- or thioester-group.

Fine chemicals thus preferably comprise organic acids such as lactic acid, succinic acid, tartaric acid, itaconic acid etc. Fine chemicals further comprise amino acids, purine and pyrimidine bases, nucleotides, lipids, saturated and unsaturated fatty acids such as arachidonic acid, alcohols, e.g. diols such as propandiol and butandiol, carbohydrates such as hyaluronic acid and trehalose, aromatic compounds such as vanillin, vitamins and cofactors etc.

A particularly preferred group of fine chemicals for the purposes of the present invention are biosynthetic products being selected from the group comprising organic acids, proteins, amino acids, lipids etc. Other particularly preferred fine chemicals are selected from the group of sulphur containing compounds such as thionine, cysteine, homocysteine, cystathionine, glutathione, biotine, thiamine and/or lipoic acid.

The group of most preferred fine chemical products include amino acids among which glycine, lysine, methionine, cysteine and threonine are particularly preferred.

In a preferred embodiment of the present invention the method for increasing the amount of a polypeptide in *C*. *glutamicum* uses modified nucleotide sequences for which codon usage has been optimized as described above and which have been derived from starting nucleotide sequences selected from the group comprising sequences encoding aspartate kinase, aspartate-semialdehyde-dehydrogenase, diaminopimelate- dehydrogenase, diaminopimelate-decarboxylase, dihydrodipicolinate-synthetase, dihydridipicolinate-reductase, pyruvate carboxylase, transcriptional regulators LuxR, transcriptional regulators LysR1, transcriptional regulators LysR2, malate-quinone-oxodoreductase, glucose-6-phosphate-deydrognease, 6-phosphogluconate-dehydrogenaase, transketolase, transaldolase, lysine-exporter, arginyl-t-RNA-synthetase, phosphoenolpyruvate-carboxylase, fructose-1,6-bisphosphatase, protein OpcA, 1-phosphofructokinase, 6-phosphofructokinase, biotin-ligase, tetrahydropicolinat-succinylase, succinyl-aminoketopimelate-aminotransferase, succinyl-diaminopimelate-desuccinylase, diaminopimelate-epimerase, 6-phosphogluconate-dehydrogenase, gucosephosphate-isomerase, phosphoglycerate-mutase, pyruvate-kinase, aspartate-transaminase and malate-enzyme.

In a particularly preferred embodiment of the present invention the method for increasing the amount of a polypeptide in a *C*. *glutamicum* uses modified nucleotide sequences for which codon usage has been optimized as described above and which have been derived from starting nucleotide sequences selected from the group comprising sequences encoding aspartate-kinase, aspartate-semialdehyde-dehydrogenase, homoserine-dehydrogenase, glycerinaldehyde-3-phosphate-dehydrogenase, 3-phosphoglycerate-kinase, pyruvate-carboxylase, homoserine-O-ccetyltransferase, cystahionine-gamma-synthase, cystahionine-beta-lyase, serine-hydroxymethyltransferase, O-acetylhomoserine-sulfhydrylase, methylene-tetrahydrofolate-reductase, phosphoserine-aminotransferase, phosphoserine-phosphatase, serine-acetyl-transferase, cysteine-synthase, cysteine-synthase II, coenzyme B12-dependent methionine-synthase (metH), coenzym B12-independent methionine-synthase, sulfate-adenylyltransferase, phosphoadenosins-phosphosulfate-reductase, ferredoxine-sulfite-reductase, ferredoxine-NADPH-reductase, ferredoxine-protein activity of sulfate-reduction RXA077, protein activity of sulfate-reduction RXA248, protein activity of sulfate-reduction RXA247, protein activity of RXA655-regulator and protein activity of RXN2910-regulator, 6-phosphogluconate-dehydrogenase, glucosephosphate-isomerase, phosphoglycerate-mutase, pyruvate-kinase, aspartate-transaminase, malate-enzyme, dihydrodipicolinate-synthetase, dihydridipicolinate-reductase, diaminopimelate- dehydrogenase, diaminopimelate-decarboxylase, lysine-exporter, pyruvate carboxylase, phosphoenolpyruvate (PEP) carboxylase, glucose-6-phosphate-deydrognease, 6-phospho-gluconolactonase, ribose-5-phosphate-isomerase, ribose-phosphate epimerase, transketolase, transaldolase, glucosephosphate-isomerase, transcriptional regulators LuxR, transcriptional regulators LysR1, transcriptional regulators LysR2, malate-quinone-oxidoreductase, malate dehydrogenase, fructose-1,6-bisphosphatase triosephosphate isomease, glyceraldehyde-phosphate. dehydrogenase, phosphoglycerate kinase, phosphglycerate mutase, enolase, pyruvate kinase, arginyl-t-RNA-synthetase, protein OpcA, 1-phosphofructokinase, 6-phosphofructokinase, biotin-ligase, isocitrate lyase, malate synthase, tetrahydropicolinat-succinylase, succinyl-aminoketopimelate-aminotransferase, succinyl-diaminopimelate-desuccinylase, diaminopimelate-epimerase, aspartate-transaminase, components of the PTS sugar uptake system, accBC (acetyl CoA carboxylase), accDA (acetyl CoA carboxylase), aceA (isocitrat-lyase), acp (acyl carrier protein), asp (aspartase), atr61 (ABC transporter), ccsB (cytochrom c synthesis protein), cdsA (phosphatidat-cytidylyltransferase), citA (sensor kinase of a 2-component system), cls (cardiolipin synthase), cma (cyclopropane-myolic acid synthase), cobW (cobalamin synthesis-related protein), cstA (carbon starvation protein A), ctaD (Cytocrom aa3 Oxidase UE1), ctaE (cytocrom aa3 oxidase UE3), ctaF,4 (subunit of cytochrome aa3 oxidase), cysD (sufate-adenosyltransferase), cysE (serine-acetyltransferase, cysH, cysK (cysteine synthase), cysN (sulfat-adenosyltransferase), cysQ (ransport protein), dctA (C4 dicarboxylate transport protein), dep67 (cobalamin synthesis-related protein), dps (DNA protection protein), dtsR (propionyl-CoA carboxylase), fad 15 (acyl-CoA-synthase), ftsX (cell division protein), glbO (HB-like protein), glk (glukokinase), gpmB (phosphoglycerate kinase II), hemD hemB (uroporphyrinogen-II-synthase, delta-aminolevulinic acid dehydratase), Ildd2 (lactate dehydrogenase), metY (O-acetylhomoserine-sulfhydrylase), msiK (sugar import protein), ndkA (nucleoside diphosphate kinase), nuoU (NADH-dehydrogenase subunit U), nuoV (NADH-dehydrogenase subunit V), nuoW (NADH-dehydrogenase subunit W), oxyR (transcriptional regulator), pgsA2 (CDP-diacylglycerol-3-P-3-phosphatidyltransferase), pknB (protein kinase B), pknD (protein kinase D), plsC (1-Acyl-SN-glycerol-3-P-acyltransferase), poxB gnd (pyruvat oxidase, 6-phosphogluconate dehydrogenase), ppgK (polyphosphate glucokinase) ppsA (PEP synthase), qcrA (Rieske Fe-S-protein), qcrA (Rieske Fe-S-protein), qcrB (cytochrom B), qcrB (cytochrom B), qcrC (cytochrom C), rodA (cell division protein), rpe (ribulose phosphate isomerase), rpi (phosphopentose isomerase), sahH (adenosyl homocysteinase), sigC (sigma factor C), sigD (activator of transcrption factor sigma D), sigE (sigma factor E), sigh (sigma factor H), sigM (sigma factor M), sod (superoxiddismutase), thyA (thymidylate synthase), truB (tRNA pseudouridine 55 synthase) and zwal(PS1-protein). These sequences and methods may be used to particularly obtain lysine.

In a further particularly preferred embodiment of the present invention the method for increasing the amount of a polypeptide in *C. glutamicum* uses modified nucleotide sequences for which codon usage has been optimized as described above and which have been derived from starting nucleotide sequences selected from the group comprising sequences encoding aspartate-kinase, aspartate-semialdehyde-dehydrogenase, glycerinaldehyde-3 -phosphate-dehydrogenase, 3-phosphoglycerate-kinase, pyruvate-carboxylase, triosephosphate-isomerase, threonine-synthase, threonin-export-carrier, transaldolase, transketolase, glucose-6-phosphate-dehydrogenase, malate-qinone-oxidoreductase, homoserine-kinase, biotine-ligase, phosphoenolpyruvate-carboxylase, threonine-efflux activity, protein OpcA, 1-phosphofructo-kinase, 6-phosphofructo-kinase, fructose-1,6-bisphosphatase, 6-phosphogluconate-dehydrogenase, homoserine-dehydrogenase 6-phosphogluconate-dehydrogenase, phosphoglycerate-mutase, pyruvat-kinase, aspartate-transaminase and malate-enzyme. These sequences and methods may be particularly be suited to obtain methionine.

In a further particularly preferred embodiment of the present invention the method for increasing the amount of a polypeptide in *C*. *glutamicum* uses modified nucleotide sequences for which codon usage has been optimized as described above and which have been derived from starting nucleotide sequences selected from the group comprising sequences encoding dehydratase, homoserin O-ccetyltransferase, serine-hydroxymethyltransferase, O-acetylhomoserine-sulfhydrylase, meso-siaminopimelate-D-dehydrogenase, phosphoenolpyruvate-carboxykinase, pyruvat-oxidase, dihydrodipicolinate-synthetase, dihydrodipicolinate-reductase, asparaginase, aspartate-decarboxylase, lysine-exporter, acetolactate-synthase, ketol-aid-reductoisomerase, branched chain aminotransferase, coenzyme B12-dependent methionine-synthase (metH), coenzym B12-independent methionine-synthase, dihydroxy acid-dehydratase and diaminopicolinate-decarboxylase. These sequences and methods may be particularly be suited to obtain threonine.

Another aspect of the present disclosure relates to the recombinant modified nucleotide sequences which encode for a polypeptide allowing for increased expression of the polypeptide in a host cell wherein the modified nucleotide sequence is derived from a different starting nucleotide sequence with the codon usage of the modified nucleotide sequence being adjusted to the codon usage of the abundant proteins of the respective host cells. The modified and starting nucleotide sequence encode for the same amino acid sequence. Both sequences encode for identical amino acid sequences.

The definitions given above as to the meaning of host cell, abundant proteins and how to determine them equally apply.

Preferred embodiments also relate to modified nucleotide sequences which are to be used for expression of a polypeptide in the host cell and wherein the modified nucleotide sequences have been derived from a starting sequence by adjusting the codon usage of the modified nucleotide sequence to the codon usage of abundant proteins of *C. glutamicum.* The modified and starting nucleotide sequence encode for the same amino acid sequence. Both sequences encode for identical amino acid sequences.

Again, for the purposes of this aspect of the invention, the definitions given above equally apply so that the term abundant proteins in e.g. *C*. *glutamicum* will essentially relate to the same group of fourteen proteins mentioned above.

In addition or alternatively, the modified nucleotide sequences for expression of polypeptides in *C. glutamicum* may use the codons GUU for valine, GCU for alanine, GAC for aspartic acid, GAG for glutamic acid and/or ATG for the start methionine.

A particularly preferred embodiment of the present invention relates to a modified nucleotide sequence that is used to drive expression of a polypeptide in *C*. *glutamicum* wherein the codons of the modified nucleotide sequence have been selected for at least one and preferably for each amino acid from the codon usage of Table 3.

In all of the aforementioned modified nucleotide sequences for increasing expression of a polypeptide in *C*. *glutamicum,* rare codons, very rare codons and extremely rare codons are preferably exchanged by the most frequently used codons as they can be taken from Table 2.

These modified nucleotide sequences may again be preferably selected from the group comprising nucleotide sequences encoding genes of biosynthetic pathways of fine chemicals. The definitions and preferences as to the meaning and desirability of fine chemicals given above equally apply.

As a consequence, in the case of producing the fine chemical lysine the nucleotide sequence may be selected from the group comprising the aforementioned sequences. The same applies if the fine chemicals methionine and threonine are to be produced.

Further aspects of the disclosure relate to vectors that are suitable for expression of a polypeptide in a host cell wherein the vector comprises the aforementioned nucleotide sequences. Of course, a preferred embodiment will relate to vectors that are capable of driving expression of polypeptides in *C*. *glutamicum.*

Host cells comprising the aforementioned nucleotide sequences or vectors also form part of the disclosure with host cells derived from *C*. *glutamicum* being preferred.

Other aspects of the disclosure relate to the use of methods as put forward above, to the use of nucleotide sequences as put forward above, to the use of a vector as put forward above and to the use of a host cell as put forward above for producing the aforementioned fine chemicals.

In preferred embodiments of the invention, one will use the codon usage optimised nucleotide sequences that allow to drive expression in *C*. *glutamicum* and that have been codon-usage optimised with respect to the abundant proteins of e.g. *C*. *glutamicum.*

In general, the person skilled in the art is familiar with designing constructs such as vectors for driving expression of a polypeptide in microorganisms such as *E. coli* and *C*. *glutamicum.* The person skilled in the art is also well acquainted with culture conditions of microorganisms such as *C. glutamicum* and *E. coli* as well as with procedures for harvesting and purifying fine chemicals such as amino acids and particularly lysine, methionine and threonine from the aforementioned microorganisms. Some of these aspects will be set out in further detail below.

The person skilled in the art is also well familiar with techniques that allow to change the original starting nucleotide sequence into a modified nucleotide sequence encoding for polypeptides of identical amino acid sequence but with different codon usage. This may e.g. be achieved by polymerase chain reaction based mutagenesis techniques, by commonly known cloning procedures, by chemical synthesis etc. Some of these procedures are set out in the examples.

Another embodiment of the present disclosure relates to a method of increasing the amount of a polypeptide in a host cell by expressing a modified nucleotide sequence which has been amended with respect to the codon usage of the abundant proteins of the host cell.

However, other than described above where codons have been replaced in the original different non-modified sequence by selecting the more frequently used codons of the group of abundant proteins of the host cell, the modified sequences are not optimised in this way, but rather are obtained by replacing codons in the original different non-modified nucleotide sequences with codons that are used in the group of abundant proteins at a similar distribution frequency. If for example the original nucleotide sequence uses the codon CUU at a frequency of 10% and the codon CUA at a frequency of 50% and if e.g. in the group of abundant proteins the codon CUC is used at a frequency of 20% and the codon CUG is used at a frequency of 60%, in the modified nucleotide sequence the codon CUU will be replaced by CUC and the codon CUA will be replaced by CUG. Thus, in one embodiment of the present disclosure methods for increasing the amount of a polypeptide in a host cell does not aim that much at optimising coding usage in terms of overall frequency but instead of harmonising the distribution frequency throughout the coding sequence.

Yet another embodiment of the present disclosure relates to methods for increasing the amount of polypeptides in *C. glutamicum* in which a modified nucleotide sequence is expressed wherein the sequence of the modified nucleotide sequence has been adjusted to the codon usage of the complete organism of *C*. *glutamicum* as set forth in Table 1. Of course, in methods relating to this aspect of the disclosure a modified nucleotide sequence may be used wherein at least one, at least two, at least three, at least four, at least five, at least six, at least seven, at least eight, at least nine, at least ten, preferably at least 1%, at least 2%, at least 4%, at least 6%, at least 8%, at least 10%, more preferably at least 20%, at least 40%, at least 60%, at least 80%, even more preferably at least 90% or least 95% and most preferably all of the codons of the starting nucleotide sequence are replaced in the resulting modified nucleotide sequence by more frequently and preferably the most frequently used codons for the respective amino acid according to Table 1. In an even more preferred embodiment the afore-mentioned number of codons to be replaced refers to rare, very rare and particularly extremely rare codons.

The above given definitions of "modified nucleotide sequence", "starting/non-modified nucleotide sequences", "host cells" etc. as well as the explanations given e.g. for the achievable extent of expression equally apply if modification is based on the codon usage as determined for the whole organism. The definitions of rare, very rare and extremely rare as well as of frequent, very frequent and extremely frequent codons equally apply except that the relative frequency of a codon is not determined on the basis of abundant proteins but on the basis of the codon usage of the organism. Of course, the present disclosure also relates to modified nucleotide sequences the codon usage of which has been adjusted to the codon usage of the organism of *C*. *glutamicum* as put forward in Table 1. Similarly, the present disclosure relates to expression vectors which can be used to express such nucleotide sequences in *C*. *glutamicum* and host cells comprising such sequences and vectors. The host cells can be selected from the *C*. *glutamicum* strains as mentioned above. As far as optimisation of codon usage is based on *C*. *glutamicum* as an organism, the present disclosure also relates to the use of such methods, modified nucleotide sequences, vectors and host cells for producing fine chemicals. The production of fine chemicals such a amino acids and particularly lysine, methionine and tryptophane is preferred in this context. For the production of these fine chemicals, the starting nucleotide sequences may be selected from factors which are involved in the biosynthesis of these compounds and particularly from the above mentioned lists.

In the following, it will be described and set out in detail how genetic manipulations in microorganisms such as *E. coli* and particularly *Corynebacterium glutamicum* can be performed.

### Vectors and Host Cells

One aspect of the disclosure pertains to vectors, preferably expression vectors, containing a modified nucleotide sequences as mentioned above. As used herein, the term "vector" refers to a nucleic acid molecule capable of transporting another nucleic acid to which it has been linked.

One type of vector is a "plasmid", which refers to a circular double stranded DNA loop into which additional DNA segments can be ligated. Another type of vector is a viral vector, wherein additional DNA segments can be ligated into the viral genome.

Certain vectors are capable of autonomous replication in a host cell into which they are introduced (e.g., bacterial vectors having a bacterial origin of replication and episomal mammalian vectors). Other vectors (e. g., non-episomal mammalian vectors) are integrated into the genome of a host cell upon introduction into the host cell, and thereby are replicated along with the host genome. Moreover, certain vectors are capable of directing the expression of genes to which they are operatively linked.

Such vectors are referred to herein as "expression vectors".

In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e. g., replication defective retroviruses, adenoviruses and adeno-associated viruses), which serve equivalent functions.

The recombinant expression vectors of the disclosure may comprise a modified nucleic acid as mentioned above in a form suitable for expression of the respective nucleic acid in a host cell, which means that the recombinant expression vectors include one or more regulatory sequences, selected on the basis of the host cells to be used for expression, which is operatively linked to the nucleic acid sequence to be expressed.

Within a recombinant expression vector, "operably linked" is intended to mean that the nucleotide sequence of interest is linked to the regulatory sequence (s) in a manner which allows for expression of the nucleotide sequence (e.g., in an in vitro transcription/translation system or in a host cell when the vector is introduced into the host cell). The term "regulatory sequence" is intended to include promoters, repressor binding sites, activator binding sites, enhancers and other expression control elements (e.g., terminators, polyadenylation signals, or other elements of mRNA secondary structure). Such regulatory sequences are described, for example, in Goeddel; Gene Expression Technology: Methods in Enzymology 185, Academic Press, San Diego, CA (1990). Regulatory sequences include those which direct constitutive expression of a nucleotide sequence in many types of host cell and those which direct expression of the nucleotide sequence only in certain host cells. Preferred regulatory sequences are, for example, promoters such as cos-, tac-, trp-, tet-, trp-, tet-, lpp-, lac-, lpp-lac-, lacIq-, T7-, T5-, T3-, gal-, trc-, ara-, SP6-, arny, SP02, e-Pp- ore PL, SOD, EFTu, EFTs, GroEL, MetZ (all from *C. glutamicum),* which are used preferably in bacteria. Additional regulatory sequences are, for example, promoters from yeasts and fungi, such as ADC1, MFa, AC, P-60, CYC1, GAPDH, TEF, rp28, ADH, promoters from plants such as CaMV/35S, SSU, OCS, lib4, usp, STLS1, B33, nos or ubiquitin-or phaseolin-promoters. It is also possible to use artificial promoters. It will be appreciated by one of ordinary skill in the art that the design of the expression vector can depend on such factors as the choice of the host cell to be transformed, the level of expression of protein desired, etc. The expression vectors of the disclosure can be introduced into host cells to thereby produce proteins or peptides, including fusion proteins or peptides, encoded by the above-mentioned modified nucleotide sequences.

The recombinant expression vectors of the disclosure can be designed for expression of the modified nucleotide sequences as mentioned above in prokaryotic or eukaryotic cells. For example, the modified nucleotide sequences as mentioned above can be expressed in bacterial cells such as *C*. *glutamicum* and *E. coli,* insect cells (using baculovirus expression vectors), yeast and other fungal cells (see Romanos, M. A. et al. (1992), Yeast 8: 423-488; van den Hondel, C. A. M.J. J. et al.(1991) in: More Gene Manipulations in Fungi,J. W.Bennet & L. L. Lasure, eds.,p. 396-428: Academic Press: San Diego; and van den Hondel, C. A. M. J. J. & Punt, P. J.(1991) in: Applied Molecular Genetics of Fungi, Peberdy, J. F. et al., eds., p. 1-28, Cambridge University Press: Cambridge), algae and multicellular plant cells (see Schmidt, R. and Willmitzer, L. (1988) Plant Cell Rep.: 583-586). Suitable host cells are discussed further in Goeddel, Gene Expression Technology : Methods in Enzymology 185, Academic Press, San Diego, CA (1990). Alternatively, the recombinant expression vector can be transcribed and translated in vitro, for example using T7 promoter regulatory sequences and T7 polymerase.

Expression of proteins in prokaryotes is most often carried out with vectors containing constitutive or inducible promoters directing the expression of either fusion or non-fusion proteins.

Fusion vectors add a number of amino acids to a protein encoded therein, usually to the amino terminus of the recombinant protein but also to the C-terminus or fused within suitable regions in the proteins. Such fusion vectors typically serve four purposes: 1) to increase expression of recombinant protein; 2) to increase the solubility of the recombinant protein; and 3) to aid in the purification of the recombinant protein by acting as a ligand in affinity purification 4) to provide a "tag" for later detection of the protein. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase.

Typical fusion expression vectors include pGEX (Pharmacia Biotech Inc; Smith, D. B. and Johnson, K. S. (1988) Gene 67: 31-40), pMAL (New England Biolabs, Beverly, MA) and pRIT5 (Pharmacia, Piscataway, NJ) which fuse glutathione S-transferase (GST), maltose E binding protein, or protein A, respectively.

Examples of suitable inducible non-fusion *E. coli* expression vectors include pTrc (Amann et al., (1988) Gene 69: 301-315), pLG338, pACYC184, pBR322,pUC18, pUC19, pKC30, pRep4,pHS1, pHS2, pPLc236, pMBL24, pLG200, pUR290,pIN-III113-B1, egtll, pBdCl, and pET lld (Studier etal., Gene Expression Technology : Methods in Enzymology 185, Academic Press, San Diego, California (1990) 60-89; and Pouwels et al., eds. (1985) Cloning Vectors. Elsevier: New York IBSN 0 444 904018). Target gene expression from the pTrc vector relies on host RNA polymerase transcription from a hybrid trp-lac fusion promoter. Target gene expression from the pET lld vector relies on transcription from a T7 gnlO-lac fusion promoter mediated by a coexpressed viral RNA polymerase (T7gnl). This viral polymerase is supplied by host strains BL21 (DE3) or HMS174 (DE3) from a resident X prophage harboring a T7gnl gene under the transcriptional control of the lacUV 5 promoter. For transformation of other varieties of bacteria, appropriate vectors may be selected. For example, the plasmids pIJ101, pIJ364, pIJ702 and pIJ361 are known to be useful in transforming Streptomyces, while plasmidspUB110, pC194 or pBD214 are suited for transformation of Bacillus species. Several plasmids of use in the transfer of genetic information into Corynebacterium include pHM1519, pBL1, pSA77 or pAJ667 (Pouwels et al., eds. (1985) Cloning Vectors. Elsevier: New York IBSN 0 444 904018).

Examples of suitable *C*. *glutamicum* and *E coli* shuttle vectors are e.g. pClik5aMCS (WO2005059093) or can be found in Eikmanns et al (Gene. (1991) 102, 93-8).

Examples for suitable vectors to manipulate Corynebacteria can be found in the Handbook of Corynebacterium (edited by Eggeling and Bott, ISBN 0-8493-1821-1, 2005). One can find a list of *E. coli - C. glutamicum* shuttle vectors (table 23.1), a list of *E. coli - C. glutamicum* shuttle expression vectors (Table 23.2), a list of vectors which can be used for the integration of DNA into the *C. glutamicum* chromosome (Table 23.3), a list of expression vectors for integration into the *C*. *glutamicum* chromosome (Table 23.4.) as well as a list of vectors for site-specific integration into the *C*. *glutamicum* chromosome (Table 23.6).

In another embodiment of the disclosure, the protein expression vector is a yeast expression vector. Examples of vectors for expression in yeast S. cerevisiae include pYepSecl (Baldari, et al., (1987) Embo J. 6: 229-234)" 2i, pAG-1, Yep6, Yep13, pEMBLYe23, pMFa (Kurjan and Herskowitz, (1982) Cell 30: 933-943), pJRY88 (Schultz et al., (1987) Gene 54: 113-123), and pYES2 (Invitrogen Corporation, San Diego, CA). Vectors and methods for the construction of vectors appropriate for use in other fungi, such as the filamentous fungi, include those detailed in: van den Hondel, C. A. M. J. J. & Punt,P. J. (1991) in: Applied Molecular Genetics of Fungi, J. F. Peberdy, et al., eds., p. 1-28, Cambridge University Press: Cambridge, and Pouwels et al., eds. (1985) Cloning Vectors. Elsevier: New York (IBSN 0 444 904018).

For the purposes of the present invention, an operative link is understood to be the sequential arrangement of promoter, coding sequence, terminator and, optionally, further regulatory elements in such a way that each of the regulatory elements can fulfill its function, according to its determination, when expressing the coding sequence.

In another embodiment of the disclosure, the modified nucleotide sequences as mentioned above may be expressed in unicellular plant cells (such as algae) or in plant cells from higher plants (e. g., the spermatophytes, such as crop plants). Examples of plant expression vectors include those detailed in: Becker, D., Kemper, E., Schell, J. and Masterson, R. (1992) Plant Mol. Biol. 20: 1195-1197; and Bevan, M. W. (1984) Nucl. Acid. Res. 12: 8711-8721, and include pLGV23, pGHlac+, pBIN19, pAK2004, and pDH51 (Pouwels et al., eds. (1985) Cloning Vectors. Elsevier: New York IBSN 0 444 904018).

For other suitable expression systems for both prokaryotic and eukaryotic cells see chapters 16 and 17 of Sambrook, J. et al. Molecular Cloning: A Laboratory Manual. 3rd ed., Cold Spring Harbor Laboratory, Cold Spring Harbor Laboratory Press, Cold Spring Harbor, NY, 2003.

In another embodiment of the disclosure, the recombinant mammalian expression vector is capable of directing expression of the nucleic acid preferentially in a particular cell type, e.g. in plant cells (e. g., tissue-specific regulatory elements are used to express the nucleic acid). Tissue-specific regulatory elements are known in the art.

Another aspect of the disclosure pertains to organisms or host cells into which a recombinant expression vector of the disclosure has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

Vector DNA can be introduced into prokaryotic or eukaryotic cells via conventional transformation or transfection techniques. As used herein, the terms "transformation" and "transfection", "conjugation" and "transduction" are intended to refer to a variety of art-recognized techniques for introducing foreign nucleic acid (e. g., linear DNA or RNA (e. g., a linearized vector or a gene construct alone without a vector) or nucleic acid in the form of a vector (e.g., a plasmid, phage, phasmid, phagemid, transposon or other DNA) into a host cell, including calcium phosphate or calcium chloride co-precipitation, DEAE-dextran-mediated transfection, lipofection, natural competence, conjugation chemical-mediated transfer, or electroporation. Suitable methods for transforming or transfecting host cells can be found in Sambrook, et al. (Molecular Cloning : A Laboratory Manual. 3rd ed., Cold Spring Harbor Laboratory, Cold Spring Harbor Laboratory Press, Cold Spring Harbor, NY, 2003), and other laboratory manuals.

In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin, kanamycine, tratracycleine, ampicillin and methotrexate. Nucleic acid encoding a selectable marker can be introduced into a host cell on the same vector as that encoding the above-mentioned modified nucleotide sequences or can be introduced on a separate vector. Cells stably transfected with the introduced nucleic acid can be identified by drug selection (e. g., cells that have incorporated the selectable marker gene will survive, while the other cells die).

When plasmids without an origin of replication and two different marker genes are used (e.g. pClik int sacB), it is also possible to generate marker-free strains which have part of the insert inserted into the genome. This is achieved by two consecutive events of homologous recombination (see also Becker et al., APPLIED AND ENVIRONMENTAL MICROBIOLOGY, 71 (12), p. 8587-8596). The sequence of plasmid pClik int sacB can be found in WO2005059093; SEQ ID 24; the plasmid is called pCIS in this document).

In another embodiment, recombinant microorganisms can be produced which contain selected systems which allow for regulated expression of the introduced gene. For example, inclusion of one of the above-mentioned optimized nucleotide sequences on a vector placing it under control of the lac operon permits expression of the gene only in the presence of IPTG. Such regulatory systems are well known in the art.

In one embodiment of the disclosure, the method comprises culturing the organisms of the disclosure (into which a recombinant expression vector or into which genome has been introduced a gene comprising the modified nucleotide sequences as mentioned above) in a suitable medium for fine chemical production. In another embodiment, the method further comprises isolating the fine chemical from the medium or the host cell.

*Growth of Escherichia coli and Corynebacterium glutamicum-Media and Culture Conditions*

The person skilled in the art is familiar with the cultivation of common microorganisms such as *C.glutamicum* and *E.coli.* Thus, a general teaching will be given below as to the cultivation of *C.glutamicum.* Corresponding information may be retrieved from standard textbooks for cultivation of *E. coli.*

*E. coli* strains are routinely grown in MB and LB broth, respectively (Follettie et al. (1993) J. Bacteriol. 175, 4096-4103). Minimal media for E. coli is M9 and modified MCGC (Yoshihama et al. (1985) J. Bacteriol. 162,591-507), respectively. Glucose may be added at a final concentration of 1%. If appropriate, antibiotics may be added in the following amounts (micrograms per millilitre): ampicillin, 50; kanamycin, 25; nalidixic acid, 25. Amino acids, vitamins, and other supplements may be added in the following amounts: methionine, 9.3 mM; arginine, 9.3 mM; histidine, 9.3 mM; thiamine, 0.05 mM. *E. coli* cells are routinely grown at 37 C, respectively.

Genetically modified *Corynebacteria* are typically cultured in synthetic or natural growth media. A number of different growth media for *Corynebacteria* are both well-known and readily available (Liebl et al. (1989) Appl.Microbiol. Biotechnol., 32: 205-210; von der Osten et al. (1998) Biotechnology Letters, 11: 11-16; Patent DE 4,120,867; Liebl(1992) "The Genus Corynebacterium, in: The Procaryotes, Volume II, Balows, A. et al., eds. Springer-Verlag) or Handbook of Corynebacterium glutamicum (2005) ISBN 0-8493-1821-1).

These media consist of one or more carbon sources, nitrogen sources, inorganic salts, vitamins and trace elements. Preferred carbon sources are sugars, such as mono-, di-, or polysaccharides. For example, glucose, fructose, mannose, galactose, ribose, sorbose, ribose, lactose, maltose, sucrose, glycerol, raffinose, starch or cellulose serve as very good carbon sources.

It is also possible to supply sugar to the media via complex compounds such as molasses or other by-products from sugar refinement. It can also be advantageous to supply mixtures of different carbon sources. Other possible carbon sources are alcohols and organic acids, such as methanol, ethanol, acetic acid or lactic acid. Nitrogen sources are usually organic or inorganic nitrogen compounds, or materials which contain these compounds. Exemplary nitrogen sources include ammonia gas or ammonia salts, such as NH₄Cl or (NN₄)₂S0₄, NH₄0H, nitrates, urea, amino acids or complex nitrogen sources like corn steep liquor, soy bean flour, soy bean protein, yeast extract, meat extract and others.

The overproduction of methionine is possible using different sulfur sources. Sulfates, thiosulfates, sulfites and also more reduced sulfur sources like H₂S and sulfides and derivatives can be used. Also organic sulfur sources like methyl mercaptan, thioglycolates, thiocyanates, and thiourea, sulfur containing amino acids like cysteine and other sulfur containing compounds can be used to achieve efficient methionine production. Formate may also be possible as a supplement as are other C1 sources such as methanol or formaldehyde.

Inorganic salt compounds which may be included in the media include the chloride-, phosphorous-or sulfate-salts of calcium, magnesium, sodium, cobalt, molybdenum, potassium, manganese, zinc, copper and iron. Chelating compounds can be added to the medium to keep the metal ions in solution. Particularly useful chelating compounds include dihydroxyphenols, like catechol or protocatechuate, or organic acids, such as citric acid. It is typical for the media to also contain other growth factors, such as vitamins or growth promoters, examples of which include biotin, riboflavin, thiamine, folic acid, nicotinic acid, pantothenate and pyridoxine. Growth factors and salts frequently originate from complex media components such as yeast extract, molasses, corn steep liquor and others. The exact composition of the media compounds depends strongly on the immediate experiment and is individually decided for each specific case. Information about media optimization is available in the textbook "Applied Microbiol. Physiology, A Practical Approach (Eds. P. M. Rhodes, P.F. Stanbury, IRL Press (1997) pp. 53-73, ISBN 0 19 963577 3). It is also possible to select growth media from commercial suppliers, like standard 1 (Merck) or BHI (grain heart infusion, DIFCO) or others.

All medium components should be sterilized, either by heat (20 minutes at 1.5 bar and121 C) or by sterile filtration. The components can either be sterilized together or, if necessary, separately.

All media components may be present at the beginning of growth, or they can optionally be added continuously or batch wise. Culture conditions are defined separately for each experiment.

The temperature should be in a range between 15°C and 45°C. The temperature can be kept constant or can be altered during the experiment. The pH of the medium may be in the range of 5 to 8.5, preferably around 7.0, and can be maintained by the addition of buffers to the media. An exemplary buffer for this purpose is a potassium phosphate buffer. Synthetic buffers such as MOPS, HEPES, ACES and others can alternatively or simultaneously be used. It is also possible to maintain a constant culture pH through the addition of NaOH or NH₄OH during growth. If complex medium components such as yeast extract are utilized, the necessity for additional buffers may be reduced, due to the fact that many complex compounds have high buffer capacities. If a fermentor is utilized for culturing the microorganisms, the pH can also be controlled using gaseous ammonia.

The incubation time is usually in a range from several hours to several days. This time is selected in order to permit the maximal amount of product to accumulate in the broth. The disclosed growth experiments can be carried out in a variety of vessels, such as microtiter plates, glass tubes, glass flasks or glass or metal fermentors of different sizes. For screening a large number of clones, the microorganisms should be cultured in microtiter plates, glass tubes or shake flasks, either with or without baffles. Preferably 100 ml shake flasks are used, filled with 10% (by volume) of the required growth medium. The flasks should be shaken on a rotary shaker (amplitude 25 mm) using a speed-range of 100-300'rpm. Evaporation losses can be diminished by the maintenance of a humid atmosphere; alternatively, a mathematical correction for evaporation losses should be performed.

If genetically modified clones are tested, an unmodified control clone or a control clone containing the basic plasmid without any insert should also be tested. The medium is inoculated to an OD600 of 0.5-1.5 using cells grown on agar plates, such as CM plates (10g/1 glucose, 2,5g/1 NaCl, 2g/1 urea, 10g/1 polypeptone, 5g/1 yeast extract, 5g/1 meat extract, 22g/1 NaCl, 2g/1 urea, 10g/1 polypeptone, 5g/1 yeast extract, 5g/1 meat extract, 22g/1 agar, pH 6.8 with 2M NaOH) that had been incubated at 30° C. Inoculation of the media is accomplished by either introduction of a saline suspension of *C. glutamicum* cells from CM plates or addition of a liquid preculture of this bacterium.

### Definition of recombination protocol

In the following it will be described how a strain of C. *glutamicum* with increased efficiency of methionine production can be constructed implementing the findings of the above predictions. Before the construction of the strain is described, a definition of a recombination event/protocol is given that will be used in the following.

"Campbell in," as used herein, refers to a transformant of an original host cell in which an entire circular double stranded DNA molecule (for example a plasmid being based on pCLIK int sacB has integrated into a chromosome by a single homologous recombination event (a cross-in event), and that effectively results in the insertion of a linearized version of said circular DNA molecule into a first DNA sequence of the chromosome that is homologous to a first DNA sequence of the said circular DNA molecule. "Campbelled in" refers to the linearized DNA sequence that has been integrated into the chromosome of a "Campbell in" transformant. A "Campbell in" contains a duplication of the first homologous DNA sequence, each copy of which includes and surrounds a copy of the homologous recombination crossover point. The name comes from Professor Alan Campbell, who first proposed this kind of recombination.

"Campbell out," as used herein, refers to a cell descending from a "Campbell in" transformant, in which a second homologous recombination event (a cross out event) has occurred between a second DNA sequence that is contained on the linearized inserted DNA of the "Campbelled in" DNA, and a second DNA sequence of chromosomal origin, which is homologous to the second DNA sequence of said linearized insert, the second recombination event resulting in the deletion (jettisoning) of a portion of the integrated DNA sequence, but, importantly, also resulting in a portion (this can be as little as a single base) of the integrated Campbelled in DNA remaining in the chromosome, such that compared to the original host cell, the "Campbell out" cell contains one or more intentional changes in the chromosome (for example, a single base substitution, multiple base substitutions, insertion of a heterologous gene or DNA sequence, insertion of an additional copy or copies of a homologous gene or a modified homologous gene, or insertion of a DNA sequence comprising more than one of these aforementioned examples listed above).

A "Campbell out" cell or strain is usually, but not necessarily, obtained by a counter-selection against a gene that is contained in a portion (the portion that is desired to be jettisoned) of the "Campbelled in" DNA sequence, for example the *Bacillus subtilis sacB* gene, which is lethal when expressed in a cell that is grown in the presence of about 5% to 10% sucrose. Either with or without a counter-selection, a desired "Campbell out" cell can be obtained or identified by screening for the desired cell, using any screenable phenotype, such as, but not limited to, colony morphology, colony color, presence or absence of antibiotic resistance, presence or absence of a given DNA sequence by polymerase chain reaction, presence or absence of an auxotrophy, presence or absence of an enzyme, colony nucleic acid hybridization, antibody screening, *etc.* The term "Campbell in" and "Campbell out" can also be used as verbs in various tenses to refer to the method or process described above.

It is understood that the homologous recombination events that leads to a "Campbell in" or "Campbell out" can occur over a range of DNA bases within the homologous DNA sequence, and since the homologous sequences will be identical to each other for at least part of this range, it is not usually possible to specify exactly where the crossover event occurred. In other words, it is not possible to specify precisely which sequence was originally from the inserted DNA, and which was originally from the chromosomal DNA. Moreover, the first homologous DNA sequence and the second homologous DNA sequence are usually separated by a region of partial non-homology, and it is this region of non-homology that remains deposited in a chromosome of the "Campbell out" cell.

For practicality, in *C*. *glutamicum,* typical first and second homologous DNA sequence are at least about 200 base pairs in length, and can be up to several thousand base pairs in length, however, the procedure can be made to work with shorter or longer sequences. For example, a length for the first and second homologous sequences can range from about 500 to 2000 bases, and the obtaining of a "Campbell out" from a "Campbell in" is facilitated by arranging the first and second homologous sequences to be approximately the same length, preferably with a difference of less than 200 base pairs and most preferably with the shorter of the two being at least 70% of the length of the longer in base pairs. The "Campbell In and -Out-method" is described in WO2007012078

The invention will now be illustrated by means of various examples. These examples are however in no way meant to limit the invention in any way.

### EXAMPLES

In the following it will be shown how the codon usage of abundant proteins in *C*. *glutamicum* was identified. Furthermore, examples are presented which show that usage of modified nucleotide sequences which have been optimized with regard to either the codon usage of abundant proteins or the organism of *C*. *glutamicum* can be used to increase the amount of a protein in *C*. *glutamicum.* This is shown for foreign genes as well as endogenous genes.

### 1. Identification of abundant proteins in C. glutamicum

Cellular extracts were prepared from the *C*. *glutamicum* strain ATCC 13032 and of some derivatives. For this purpose, 250 mg of cell grown under standard conditions were pelleted and suspended in 750 µl lysis buffer (20 mM TRIS, 5 mM EDTA, pH 7.5) containing a protease inhibitor mix (Complete, Roche). Cell disruption was carried out at 4 °C in a mixer mill (Retsch, MM 2000) using 0.25 - 0.5 mm glass beads. Cell debris was removed by centrifugation at 22.000 rpm for 1 hour at 4 °C. Protein concentrations were determined by the Popov (Popove et a. (1975) Acta. Biol. Med. Germ, 34, 1441-1446). Cell extracts were used immediately or frozen in aliquots at -80 °C.

For 2D polyacrylamide gel electrophoresis of proteins 30 µg of crude protein extract was resuspended 450 µl of rehydration buffer (8M urea, 2M thiourea, 1% CHAPS, 20 mM DTT, 1% Ampholines 3.5 - 10) and a few grains of bromophenol blue. For isoelectric focussing precast 24 cm-IPG strips with a linear pH gradient of 4.5 to 5.5 were used in a Multiphor II isoelectric focussing unit (Amersham Biosciences). Proteins were focused using a gradient programme up to 3500 V resulting 65.000 Vh in total. Focused IPG gels were equilibrated twice for 15 minutes in a buffer containing 1.5 M Tris-HCl (pH 8.8), 6M urea, 30 % (vol/vol) glycerol, 2 % (wt/vol) sodium dodecyl sulfate, and 1 % (wt/vol) DTT. For the second equilibration step DDT was replaced by 5 % (wt/vol) iodoacetamide, and a few grains of bromophenol blue were added. The second dimension was run in sodium dodecyl sulfate-12.5 % polyacrylamide gels in an Ettan Dalt apparatus (Amersham Biosciences) as recommended by the manufacturer, and gels were subsequently silver stained (Blum et al. (1987), Electrophoresis, 8, 93-99) in a home made staining automat.

Protein spots were excised from preparative Coomassie-stained gels (300 µg total protein load each) and digested with modified trypsin (Roche, Mannheim) as described by Hermann et al. (Electrophoresis (2001), 22, 1712-1723). Mass spectrometrical identifications were performed on an LCQ advantage (Thermo Electron) after nano-HPLC separation of the peptides (LC Packings, RP18 column, length 15 cm, i.d. 75 µm), using the MASCOT software (David et al. (1999) Electrophoresis, 20,3551-3567).

Based on the 2D gel electrophoresis results of different gels 14 proteins were identified as being abundant in *C*. *glutamicum* as these proteins could be observed at high amounts in all gels. These proteins are: Elongation Factor Tu (Genbank accession no: X77034), glycerine-aldehyde-3-phosphate-dehydrogenase (Genbank accession no: BX927152, ±, nt. 289401-288397), fructose bisphosphate aldolase (Genbank accession no: BX927156, ±, nt. 134992-133958), Elongation Factor Ts (Genbank accession no: BX927154, ±, nt. 14902-14075), hypothetical protein (Genbank accession no: BX927155, ±, nt. 213489-214325), enolase (Genbank accession no: BX927150, nt. 338561-339838) peptidyl-prolyl cis-trans isomerase (Genbank accession no: BX927148, nt. 34330-34902), superoxide dismutase (Genbank accession no: AB055218) phospho-glycerate dehydrogenase (Genbank accession no: BX927151, nt. 306039-307631) SSU Rib protein S1P(Genbank accession no: BX927152, ±, nt. 26874-28334) triosephosphate-isomerase (Genbank accession no: BX927152, ±, nt. 286884-286105) isopropyl malate synthase ( Genbank accession no: X70959) butan-2,3-dioldehydrogenase (Genbank accession no: BX927156, nt. 20798-21574 ) and fumarat hydratase (Genbank accession no: BX927151, ±, nt. 18803-17394).

The coding sequences of these genes were then fed into the "Cusp" function of the EMBOSS tool box using standard parameters In an independent approach the genomic sequence of the complete *C*. *glutamicum* strain ATCC 13032 was used to generate a codon usage table for the organism as a whole.

The codon usage frequencies as determined for the aforementioned 14 abundant proteins were used to calculate codon usage frequencies for abundant proteins in *C*. *glutamicum.* The codon relative codon usage frequencies of abundant proteins in *C*. *glutamicum* are found in Table 2, while the relative codon usage frequencies of the organism as a whole are found in Table 1.

**Table 1 - Relative codon usage frequencies of Corynebacterium glutamicum ATCC 13032.**

| | | | |
|---|---|---|---|
| UUU 37.1 | UCU 17.3 | UAU 33.8 | UGU 36.5 |
| UUC 62.9 | UCC 33.6 | UAC 66.2 | UGC 63.5 |
| UUA 5.3 | UCA 13.0 | UAA 53.1 | UGA 16.7 |
| UUG 20.3 | UCG 11.9 | UAG 30.2 | UGG 100 |
| | | | |
| CUU 17,2 | CCU 23.3 | CAU 32.1 | CGU 24.5 |
| CUC 22.5 | CCC 20.2 | CAC 67.9 | CGC 44.7 |
| CUA 6.1 | CCA 34.9 | CAA 38.5 | CGA 11.8 |
| CUG 28.6 | CCG 21.6 | CAG 61.5 | CGG 8.8 |
| | | | |
| AUU 37.7 | ACU 20.4 | AAU 33.4 | AGU 7.8 |
| AUC 59.2 | ACC 52.9 | AAC 66.4 | AGC 16.4 |
| AUA 3.1 | ACA 12.5 | AAA 39.9 | AGA 4.1 |
| AUG 100 | ACG 14.2 | AAG 60.1 | AGG 6.1 |
| | | | |
| GUU 26.0 | GCU 23.7 | GAU 55.6 | GGU 30.3 |
| GUC 27.7 | GCC 25.4 | GAC 44.4 | GGC 42.4 |
| GUA 10.1 | GCA 29.3 | GAA 56.3 | GGA 18.9 |
| GUG 36.2 | GCG 21.6 | GAG 43.7 | GGG 8.4 |
| | | | |
| ATG* 72.5 | | | |
| GTG* 20.5 | | | |
| TTG* 7.0 | | | |

| | | | |
|---|---|---|---|
| *designates start codons; relative Frequencies are in percentage. | | | |

**Table 2 - Relative codon usage frequencies of 14 abundant proteins in Corynebacterium glutamicum ATCC 13032.**

| | | | |
|---|---|---|---|
| UUU 10.6 | UCU 20.2 | UAU 3.6 | UGU 25.0 |
| UUC 89.4 | UCC 65.4 | UAC 96.4 | UGC 75.0 |
| UUA 0.8 | UCA 3.3 | UAA 92.9 | UGA 0.0 |
| UUG 7.5 | UCG 2.2 | UAG 7.1 | UGG 100 |
| | | | |
| CUU 20.8 | CCU 37.6 | CAU 5.8 | CGU 39.6 |
| CUC 25.4 | CCC 4.4 | CAC 94.2 | CGC 57.6 |
| CUA 2.6 | CCA 51.4 | CAA 7.7 | CGA 2.2 |
| CUG 42.9 | CCG 6.6 | CAG 92.3 | CGG 0.6 |
| | | | |
| AUU 17.1 | ACU 18.9 | AAU 9.7 | AGU 0.8 |
| AUC 82.6 | ACC 78.9 | AAC 90.3 | AGC 8.1 |
| AUA 0.3 | ACA 1.4 | AAA 7.1 | AGA 0.0 |
| AUG 100 | ACG 0.8 | AAG 92.9 | AGG 0.0 |
| | | | |
| GUU 47.9 | GCU 46.8 | GAU 34.9 | GGU 32.3 |
| GUC 34.0 | GCC 9.9 | GAC 65.1 | GGC 59.0 |
| GUA 6.5 | GCA 35.9 | GAA 32.5 | GGA 8.2 |
| GUG 11.6 | GCG 7.4 | GAG 67.5 | GGG 0.5 |
| | | | |
| ATG* 78.6 | | | |
| GTG* 21.4 | | | |
| TTG* 0.0 | | | |

| | | | |
|---|---|---|---|
| * indicates start codons; relative Frequencies are in percentage. | | | |

Table 2 was then used to determine the codons that are used most frequently for each amino acid in the abundant proteins of *C*. *glutamicum.* This information is displayed in Table 3 below.

**Table 3 - Codon usage of 14 abundant proteins in Corynebacterium glutamicum ATCC 13032.**

| | |
|---|---|
| UUC | F |
| UCC | S |
| UAC | Y |
| UGC | C |
| UAA | Stop |
| ATG if start codon | M |
| UGG | W |
| CUG | L |
| CCA | P |
| CAC | H |
| CGC | R |
| CAG | Q |
| AUC | I |
| ACC | T |
| AAC | N |
| AUG | M |
| AAG | K |
| GUU | V |
| GCU | A |
| GAC | D |
| GGC | G |
| GAG | E |

Table 4 shows the frequencies of codons which are not calculated on the basis of codons encoding a specific amino acid, but on the basis of all codons for all amino acids. The values in brackets indicate the absolute number of the respective codon. The relative frequencies of Table 1 were calculated on the basis of these absolute numbers. The values refer to the organism of *C*. *glutamicum.*

**Table 4 - Codon usage of Corynebacterium glutamicum ATCC 13032.**

| | | | | | | | |
|---|---|---|---|---|---|---|---|
| UUU | 13.4( 25821) | UCU | 11.0( 21227) | UAU | 7.5( 14384) | UGU | 2.4( 4605) |
| UUC | 22.8( 43837) | UCC | 21.4( 41118) | UAC | 14.7( 28214) | UGC | 4.2( 8015) |
| UUA | 5.1( 9795) | UCA | 8.3( 15898) | UAA | 1. 7 ( 3272) | UGA | 0.5( 1032) |
| UUG | 19.6( 37762) | UCG | 7.6( 14639) | UAG | 1.0( 1859) | UGG | 14.1( 27072) |
| | | | | | | | |
| CUU | 16.7( 32074) | CCU | 11.3( 21668) | CAU | 6.8( 12991) | CGU | 13.7( 26310) |
| CUC | 21.8( 41988) | CCC | 9.7( 18716) | CAC | 14.3( 27445) | CGC | 24.9( 47939) |
| CUA | 5.9( 11320) | CCA | 16.9( 32429) | CAA | 13.0( 24975) | CGA | 6.6( 12698) |
| CUG | 27.7( 53261) | CCG | 10.4( 20070) | CAG | 20.7( 39864) | CGG | 4.9( 9466) |
| | | | | | | | |
| AUU | 21.7( 41804) | ACU | 12.6( 24184) | AAU | 10.9( 21056) | AGU | 4.9( 9515) |
| AUC | 34.1( 65557) | ACC | 32.5( 62592) | AAC | 21.8( 42037) | AGC | 10.4( 20019) |
| AUA | 1.8( 3483) | ACA | 7.7( 14747) | AAA | 13.9( 26703) | AGA | 2.3( 4445) |
| AUG | 22.1( 42484) | ACG | 8.8( 16879) | AAG | 20.9( 40213) | AGG | 3.3( 6398) |
| | | | | | | | |
| GUU | 20.8( 40069) | GCU | 25.4( 48864) | GAU | 33.0( 63429) | GGU | 24.3( 46678) |
| GUC | 22.2( 42696) | GCC | 27.2( 52264) | GAC | 26.4( 50716) | GGC | 34.0( 65427) |
| GUA | 8.1( 15628) | GCA | 31.3( 60329) | GAA | 35.7( 68737) | GGA | 15.2( 29219) |
| GUG | 28.9( 55708) | GCG | 23.2( 44613) | GAG | 27.7( 53381) | GGG | 6.7( 12923) |

Frequencies are indicated after the codons in /1000.

Table 5 shows the frequencies of codons which were not calculated on the basis of codons encoding a specific amino acid, but on the basis of all codons for all amino acids. The values in brackets indicate the absolute number of the respective codon. The relative frequencies of Table 2 were calculated on the basis of these absolute numbers. The values refer to the group of abundant proteins in *C*. *glutamicum.*

**Table 5 - Codon usage of 14 abundant proteins in Corynebacterium glutamicum ATCC 13032.**

| | | | | | | | |
|---|---|---|---|---|---|---|---|
| UUU | 3.6( 18) | UCU | 10.9( 55) | UAU | 0.8( 4) | UGU | 1.2( 6) |
| UUC | 30.0( 152) | UCC | 35.2( 178) | UAC | 21.1( 107) | UGC | 3.6( 18) |
| UUA | 0.6( 3) | UCA | 1.8( 9) | UAA | 2.6( 13) | UGA | 0.0( 0) |
| UUG | 5.7 ( 29) | UCG | 1.2 ( 6) | UAG | 0.2 ( 1) | UGG | 8.3 ( 42) |
| | | | | | | | |
| CUU | 16.0( 81) | CCU | 13.4 ( 68) | CAU | 1.2 ( 6) | CGU | 17.4( 88) |
| CUC | 19.6( 99) | CCC | 1.6( 8) | CAC | 19.4 ( 98) | CGC | 25.3( 128) |
| CUA | 2.0( 10) | CCA | 18.4( 93) | CAA | 2.6( 13) | CGA | 1.0( 5) |
| CUG | 33.0( 167) | CCG | 2.4( 12) | CAG | 30.6( 155) | CGG | 0.2 ( 1) |
| | | | | | | | |
| AUU | 9.9( 50) | ACU | 10.5( 53) | AAU | 4.0( 20) | AGU | 0.4( 2) |
| AUC | 47.8( 242) | ACC | 43.7( 221) | AAC | 36.8( 186) | AGC | 4.3( 22) |
| AUA | 0.2 ( 1) | ACA | 0.8( 4) | AAA | 3.6( 18) | AGA | 0.0( 0) |
| AUG | 17.2 ( 87) | ACG | 0.4( 2) | AAG | 46.4( 235) | AGG | 0.0( 0) |
| | | | | | | | |
| GUU | 43.5( 220) | GCU | 54.9( 278) | GAU | 21.9( 111) | GGU | 28.1( 142) |
| GUC | 30.8( 156) | GCC | 11.7( 59) | GAC | 40.9( 207) | GGC | 51.2( 259) |
| GUA | 5.9( 30) | GCA | 42.1( 213) | GAA | 27.9( 141) | GGA | 7.1( 36) |
| GUG | 10.5( 53) | GCG | 8.7( 44) | GAG | 57.9( 293) | GGG | 0.4( 2) |

| | | | | | | | |
|---|---|---|---|---|---|---|---|
| Frequencies are indicated after the codons in /1000. | | | | | | | |

Surprisingly there are many significant differences in the codon usage between tables generated by using all proteins (whole genome, Table 1) compared to the situation where only the above-specified abundant genes are considered (Table 2). Some of the examples are shown in Table 6 below.

**Table 6 - Relative frequency of codons used**

| Amino acid | Codon | Whole genome | Abundant proteins |
|---|---|---|---|
| q | caa | 38.5% | 7.7 |
| q | cap | 61.5% | 91.3 |
| y | tac | 66.0% | 96.4 |
| y | tat | 33.8% | 3.6 |

### 2. Reference Example: Improved protein expression of a heterologous gene in C. glutamicum

It was considered to use the above finding for optimizing gene expression of foreign heterologous genes in *C*. *glutamicum*.

To this end the coding sequence of lysine-2,3-aminomutase from *Clostridium subterminale* was used. The accession number for wild type aminomutase from *C*. *subterminale* is Q9XBQ8 (protein sequence), AF159146 (nucleotide sequence)

Introduction of the enzymatic activities of lysine-2,3 aminomutase in *C. glutamicum* is highly interesting because this enzyme catalyzes the isomerization of L-lysine into β-lysine. β-lysine as well as L-lysine may be interesting compounds as they can be used as precursor molecules in the production of ε-caprolactam which is used for industrially important polymers such as Nylon 6.

While L-lysine may also be used for ε-caprolactam synthetization via cyclization of L-lysine followed by deamination, β-lysine may be more interesting because deamination may be performed without the relatively expensive chemical hydroxylamine-O-sulfonic acid.

Furthermore, β-lysine is also a constituent of antibiotics produced by Streptomyces and Norcardia such as viomycin, streptolin A, streptothricin, roseothricin, geomycin and myomicin. It may therefore be interesting to have an organism available that is derived from *C. glutamicum* and allows for efficient production of β-lysine by catalyzing the isomerization of naturally produced L-lysine.

However, as the gene for lysine 2,3-aminomutase is not present in *C*. *glutamicum* expression of the original *C*. *subterminale* sequence may not proceed efficiently enough. For cloning of *C*. *subterminale* lysine 2,3-aminomutase the PCR primers WKJ90 (cctaacacagaaatgtc) (SEQ ID No. 3) and WKJ65 (cagtctgcatcgctaacatc) (SEQ ID No. 4) were used together with the chromosome of *C*. *subterminale* as a template to amplify a DNA fragment of up- and downstream regions including N- and C-terminal sequences of *kamA* gene respectively. The resulting amplification product was purified and subsequently the full sequence of *the C. subterminale kamA* gene which includes the gene for the aminomutase were amplified using PCR primers WKJ105 (atcttcttggcagaactcatgggtaaaaaatcctttcgta) (SEQ ID No. 5) and WKJ106 (gagagagatctagatagctgccaattattccggg) (SEQ ID No. 6). The amplified PCR fragment was purified, digested with restriction enzymes XhoI and MloI and ligated to the pClik5aMCS which had been digested with the same restriction enzymes.

As the codon usage for the *C*. *subterminale kamA* gene is quite different to that of the abundant genes of *C*. *glutamicum,* expression of the *C*. *subterminale kamA* may not be efficient in a *C*. *glutamicum* lysine producing strain.

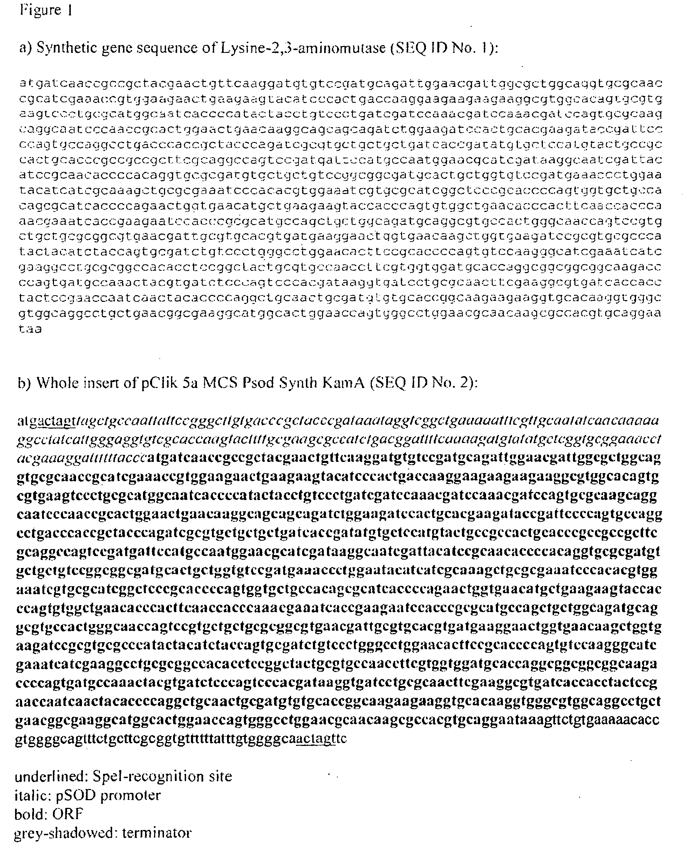

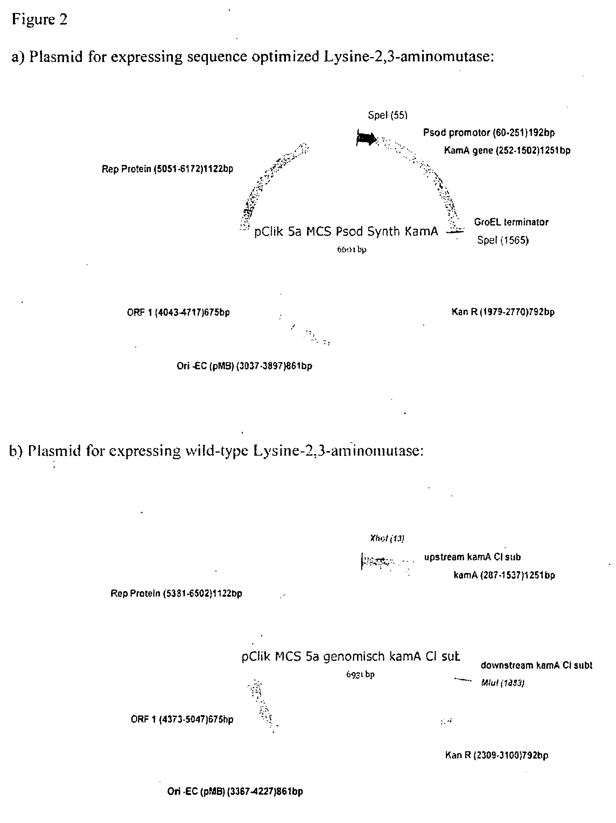

To enhance gene expression in *C*. *glutamicum,* a synthetic *kamA* gene was therefore created with the sequence of the synthetic gene being adapted to *C*. *glutamicum* codon usage on the basis of the codon usage as determined for the whole organism of *C*. *glutamicum* (SEQ ID No. 1). Furthermore, the synthetic *kamA* gene had a *C*. *glutamicum* sodA promoter (Psod) and a groEL terminator. The sequence of the synthetic *kamA* gene is shown in Fig. 1 (Seq ID No. 2). The genomic *kamA* gene was introduced into pClik using the endogenous *kamA* promoter (pClik 5a MCS genomisch kamA Cl sub, see Figure 2b). The DNA constructs used for expression of the original sequence of *C*. *subterminale* aminomutase and the synthetic gene are schematically shown in Fig. 2

Subsequently, a lysine producing strain of *C*. *glutamicum* was transformed by electroporation with recombinant plasmids harboring the aforementioned synthetic lysine 2,3-aminomutase gene or the respective wild type *C*. *subterminale* lysine 2,3-aminomutase gene. The plasmids were based on pClik. Shaking flask experiments were performed on the recombinant strains to test β-lysine production. The same culture medium and conditions were employed.

For the control, the host strain and recombinant strain having the empty plasmid pClik5aMCS were tested in parallel. The strains were precultured on CM agar at 30°C overnight. Cultured cells were then harvested in a microtube containing 1.5ml of 0.9% NaCl and cell density was determined by the absorbance at 610nm following vortex. For the main culture, suspended cells were inoculated to reach 1.5 of initial OD into 10 ml of the production medium contained in an autoclaved 100ml of Erlenmeyer flask having 0.5g of CaCO₃. In case a recombinant strain was cultured, 20µg/ml of kanamycine was added to all media. Main culture was performed on a rotary shaker with 200rpm at 30°C for 48-78hs.

For cell growth measurement, 0.1ml of culture broth was mixed with 0.9ml of 1N HCl to eliminate CaCO₃, and the absorbance at 610 nm was measured following appropriate dilution.

The concentration of β-lysine, lysine and residual sugar including glucose, fructose and sucrose were measured by HPLC method. Culture broth was centrifuged at 13,000rpm for 5min, diluted appropriately with water (if needed), filtrated with 0.22µm filter, and followed by injection onto HPLC column.

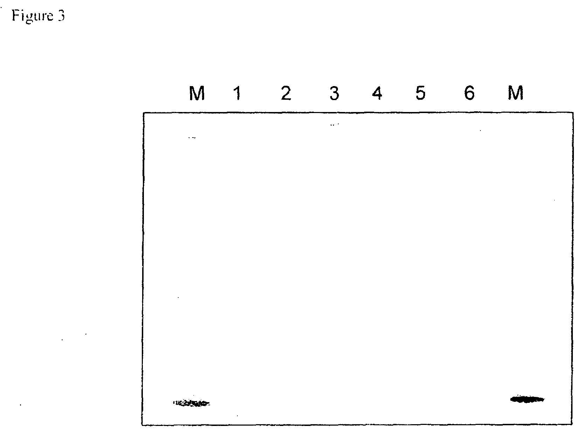

An accumulation of ß-lysine was only observed in recombinant strains containing *C*. *subterminale* synthetic *kamA* gene. In addition, expression of the genes was confirmed by SDS-PAGE (see Figure 3).

It has to be observed that the two expression constructs may not been readily comparable. The synthetic gene was expressed under the control of the strong promoter Psod which, however, is not present in the construct containing the original sequence. However, it is assumed that the increased production of β-lysine would also be observed if the synthetic gene and the original constructs were expressed under the control of identical promoters.

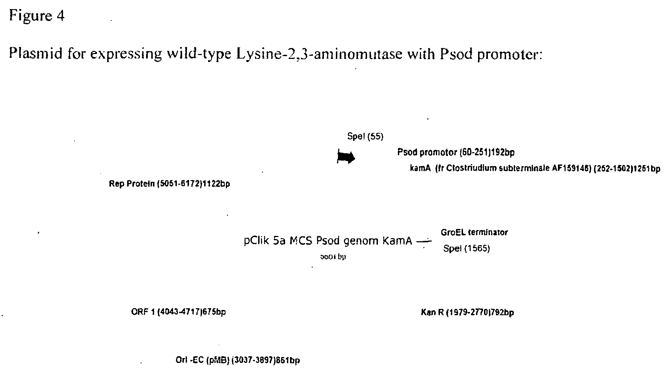

Accordingly a plasmid was constructed harbouring the genomic *kamA* gene under the control of a Psod promoter. Again the plasmid is based in pClik. A schematic representation of the resulting plasmid pClik 5a MCS Psod genom KamA (SEQ ID No. 7) is depicted in Figure 4.

The pClik 5a MCS Psod genom KamA plasmid was expressed in *C*. *glutamicum* as described above.

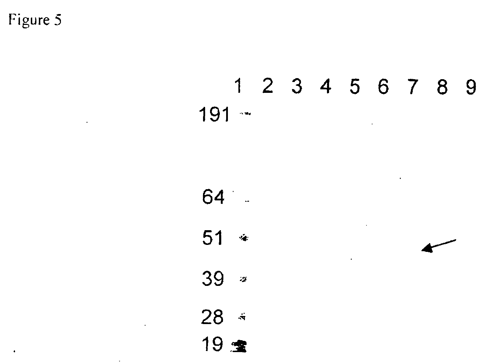

From two independent transformants, an overnight culture was grown and cell extracts were prepared. Equal amounts of total protein were loaded on an SDS-gel and the expression of the KamA protein was analyzed after Coomassie staining. As can be seen from Figure 5, the codon optimized kamA gene under control of Psod results in a high level of KamA protein while the wild type kamA sequence, also controlled by the same promoter, does not give this high level of expresseion. The expected size of KamA which is 47 kDa by an arrow. Thus, the effect of increased protein level is due to the optimization of the codon usage.

### 3. Improved protein expression of lysA in C. glutamicum

In the following it is described how to increase the amount of lysA by adapting the codon usage as mentioned above.

### 3.1 Construction of optimized lysA

The enzyme lysA is important for lysine biosynthesis. The codon usage of the coding sequence of lysA (Genbank accession no. 3344931) was determined using the Cusp function of the EMBOSS software package. The codon usage of the endogenous gene is depicted in table 7 below.

**Table 7 - codon usage of wild type lysA**

| Codon | Amino acid | Fract | /1000 | Number |
|---|---|---|---|---|
| GCA | A | 0.448 | 58.427 | 26 |
| GCC | A | 0.328 | 42.697 | 19 |
| GCG | A | 0.069 | 8.989 | 4 |
| GCT | A | 0.155 | 20.225 | 9 |
| TGC | C | 0.750 | 6.742 | 3 |
| TGT | C | 0.250 | 2.247 | 1 |
| GAC | D | 0.800 | 53.933 | 24 |
| GAT | D | 0.200 | 13.483 | 6 |
| GAA | E | 0.824 | 62.921 | 28 |
| GAG | E | 0.176 | 13.483 | 6 |
| TTC | F | 1.000 | 35.955 | 16 |
| TTT | F | 0.000 | 0.000 | 0 |
| GGA | G | 0.195 | 17.978 | 8 |
| GGC | G | 0.561 | 51.685 | 23 |
| GGG | G | 0.049 | 4.494 | 2 |
| GGT | G | 0.195 | 17.978 | 8 |
| CAC | H | 1.000 | 26.966 | 12 |
| CAT | H | 0.000 | 0.000 | 0 |
| ATA | I | 0.000 | 0.000 | 0 |
| ATC | I | 0.773 | 38.202 | 17 |
| ATT | I | 0.227 | 11.236 | 5 |
| AAA | K | 0.545 | 13.483 | 6 |
| AAG | K | 0.455 | 11.236 | 5 |
| CTA | L | 0.073 | 6.742 | 3 |
| CTC | L | 0.220 | 20.225 | 9 |
| CTG | L | 0.512 | 47.191 | 21 |
| CTT | L | 0.098 | 8.989 | 4 |
| TTA | L | 0.000 | 0.000 | 0 |
| TTG | L | 0.098 | 8.989 | 4 |
| ATG | M | 1.000 | 13.483 | 6 |
| AAC | N | 0.750 | 26.966 | 12 |
| AAT | N | 0.250 | 8.989 | 4 |
| CCA | P | 0.529 | 20.225 | 9 |
| CCC | P | 0.294 | 11.236 | 5 |
| CCG | P | 0.000 | 0.000 | 0 |
| CCT | P | 0.176 | 6.742 | 3 |
| CAA | Q | 0.286 | 4.494 | 2 |
| CAG | Q | 0.714 | 11.236 | 5 |
| AGA | R | 0.000 | 0.000 | 0 |
| AGG | R | 0.000 | 0.000 | 0 |
| CGA | R | 0.000 | 0.000 | 0 |
| CGC | R | 0.783 | 40.449 | 18 |
| CGG | R | 0.043 | 2.247 | 1 |
| CGT | R | 0.174 | 8.989 | 4 |
| AGC | S | 0.250 | 15.730 | 7 |
| AGT | S | 0.000 | 0.000 | 0 |
| TCA | S | 0.071 | 4.494 | 2 |
| TCC | S | 0.607 | 38.202 | 17 |
| TCG | S | 0.000 | 0.000 | 0 |
| TCT | S | 0.071 | 4.494 | 2 |
| ACA | T | 0.091 | 4.494 | 2 |
| ACC | T | 0.818 | 40.449 | 18 |
| ACG | T | 0.045 | 2.247 | 1 |
| ACT | T | 0.045 | 2.247 | 1 |
| GTA | V | 0.195 | 17.978 | 8 |
| GTC | V | 0.220 | 20.225 | 9 |
| GTG | V | 0.341 | 31.461 | 14 |
| GTT | V | 0.244 | 22.472 | 10 |
| TGG | W | 1.000 | 4.494 | 2 |
| TAC | Y | 0.929 | 29.213 | 13 |
| TAT | Y | 0.071 | 2.247 | 1 |
| TAA | * | 0.000 | 0.000 | 0 |
| TAG | * | 0.000 | 0.000 | 0 |
| TGA | * | 0.000 | 0.000 | 0 |

Frequencies are indicated as /1000.

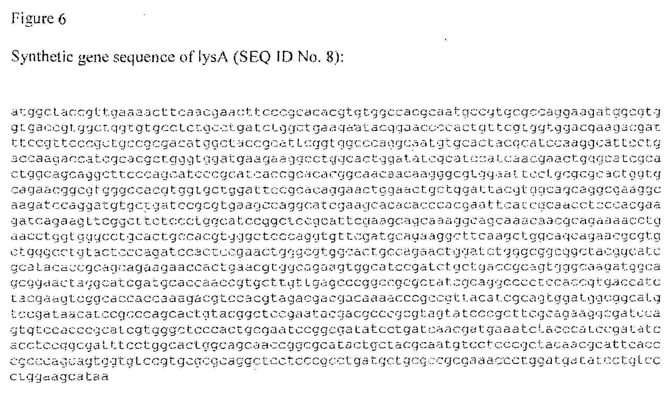

Starting from this endogenous sequence an optimized synthetic sequence was constructed. The synthetic optimized lysA sequence was provided by GeneArt GmbH (Regensburg, Germany). The sequence of the optimized lysA construct is depicted in Fig. 6 as Seq ID No. 8.

A cloning insert to be cloned into pClik int sacB was obtained containing approximately 600 (593) nucleotides upstream of the coding region of lysA, the optimized lysA coding region and approximately 600 (606) nucleotide downstream of the coding region oflysA. This construct was obtained by a set of fusion PCR based which are outlined in table 9 below.

**Table 8 - Primer sequences for lysA**

| PCR | Sense primer | Antisense primer | Template | Amplified region | Size [bp] |
|---|---|---|---|---|---|
| 1 | Old 540 | Old 541 | Genomic DNA ATCC 13032 | upstream lysA | 604 |
| 2 | Old 542 | Old 543 | Synthetic gene | cds lysA | 1377 |
| 3 | Old 544 | Old 545 | Genomic DNA ATCC 13032 | downstream lysA | 618 |
| 4 | Old 542 | Old 545 | PCR 2 and PCR 3 | Fusion cds and downstream | 1976 |
| 5 | Old 540 | Old 545 | PCR 1 and PCR 4 | Fusion upstream, cds and downstream | 2560 |

| | |
|---|---|
| Old 540 | ACTATGACGTCGGCGTTGAAGTCCTGATTGG (SEQ ID No. 12) |
| Old 541 | TGTTACATCTTCTCCGGTGC (SEQ ID No. 13) |
| Old 542 | GCACCGGAGAAGATGTAACAATGGCTACCGTTGAAAACTTCAA (SEQ ID No. 14) |
| Old 543 | GGTCAGGCGTCGAAAAGCGTTATGCTTCCAGGGACAGGA (SEQ ID No. 15) |
| Old 544 | CGCTTTTCGACGCCTGACC (SEQ ID No. 16) |
| Old 545 | ACTACTTCTAGACGACAACTCCTACTACCTCTCC (SEQ ID No. 17) |

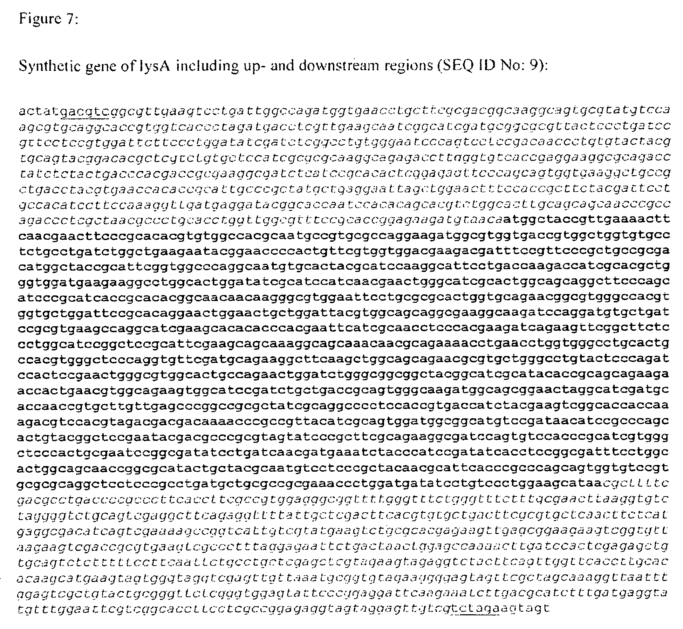

The sequence of the optimized sequence is shown in Figure 6 (SEQ ID No. 8). The sequence of the complete cloning construct is shown in Figure 7 as SEQ ID No. 9. Underlined are the Aat II and XbaI restriction sites which were introduced by the primers Old 540 and Old 545.

The PCR product was then purified, digested with Aat II and Xba I, purified again and ligated with pClik int sacB which had been linearized before with the same enzymes respectively. Integrity of the insert was confirmed by sequencing.

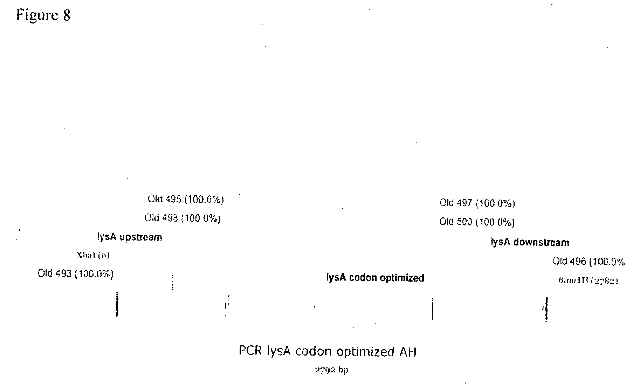

A general outline of the cloning construct is depicted in Fig. 8.

### 3.3 Construction of C. glutamicum strains containing the optimized synthetic lysA genes

The plasmid containing the optimized synthetic lysA gene can be used to replace the native coding region of the *lysA* gene by the coding region with the optimized coding usage. Two consecutive recombination events one in each of the up- and the downstream region respectively are necessary to change the complete coding sequence. The method of replacing the endogenous genes with the optimized genes is in principle described in the publication by Becker et al. (*vide supra*). The most important steps are:
- Introduction of the plasmids in the strain by electroporation. The step is e.g. described in DE 10046870 which is incorporated by reference as far as introduction of plasmids into strains is disclosed therein.
- Selection of clones that successfully have integrated the plasmid after a first homologous recombination event into the genome. This selection is achieved by growth on kanamycine-containing agar plates. In addition to that selection step, successful recombination can be checked via colony PCR.
- By incubating a positive clone in a kanamycine-free medium a second recombination event is allowed for.
- Clones in which the vector backbone are successfully removed by way of a second recombination event are then identified by growth on sucrose -containing medium. Only those clones will survive that have lost the vector backbone comprising the SacB gene.
- Then, clones in which the two recombination events had led to replacement of the native lysA-coding region can be identified with PCR-specific primers.

For verification of successful integration of the synthetic lysA gene, a PCR analyis can be performed first. To this end the following primer pair can be used:
Old 494: AACCGTGGAAAACTTCAAC (SEQ ID No. 18)
Old 499: TCCAGGGACAGGATATCA (SEQ ID No. 19)

A PCR product of approximately 1327 bp in size is expected.

Successful manipulation can be further confirmed by Southern blotting:

### Southern Blot lysA

Probes for Southern blotting can be made by PCR using the following oligonucleotides and pClik int sacB lysA codon optimized sequence as a template:
Old494: AACCGTGGAAAACTTCAAC (SEQ ID No. 18)
Old499: TTCCAGGGACAGGATATCA (SEQ ID No. 19)

Genomic DNA of the parent strain and the clones which are selected after PCR can be prepared, digested over night with an restriction enzyme as detailed below, separated on an 1% agarose gel and blotted onto a Nylon membrane according to standard methods. Detection can be done using a commercial Kit (Amersham) following the instructions of the manufacturer. For the following digest, one would expect the indicated fragments:

| Enzyme | expected fragment size native lysA: | expected fragment size optimized lysA: |
|---|---|---|
| SalI/PstI | 1294 | 2806 |
| Bgl II/ Mlu I | 3066,4646 | 591, 363, 4284, 2475 |

The Southern Blot Analysis may be used to confirm the successful integration of the synthetic lysA gene.

As parent strains, C. *glutamicum* lysine producing strains can be used.

One may use different C. glutamicum strains for this purpose. However, it is preferred to use a *C*. *glutamicum* lysine production strain such as for ATCC13032 lysC^{fbr} or other derivatives of ATCC13032 or ATCC13286. The detailed construction of ATCC13032 lysC^{fbr} is described in patent application WO2005059093.

### 3.4 Determination of expression of optimized lysA gene and lysine production.

Once one will have obtained the *C*. *glutamicum* strain ATCC13032 lysC^{fbr} derived strains comprising the optimized lysA gene several of these clones can be selected to be investigated as to any effect on lysine productivity.

To analyze the effect of the codon usage optimized synthetic ddh or lysA genes on lysine productivity, the optimized strains are compared to lysine productivity of the parent strain.

To this effect one can grow the strains on CM-plates (10% sucrose, 10 g/l glucose, 2,5 g/l NaCl, 2 g/l urea, 10 g/l Bacto Pepton, 10 g/l yeast extract, 22 g/l agar) for two days at 30°C. Subsequently cells can be scraped from the plates and re-suspended in saline. For the main culture one can grow 10 ml of medium 1 and 0.5 g autoclave CaCo₃ in a 100 ml Erlenmeyer flask together with the cell suspension up to an OD₆₀₀ of 1.5. The cells are then grown for 48 hours on a shaker of the type Infors AJ118 (Infors, Bottmingen, Switzerland) at 220 rpm. Medium 1 has the following concentration:

**Medium 1:**

| | |
|---|---|
| 40g/l | Sucrose |
| 60g/l | Melasse (calculated on the basis of 100% sugar content) |
| 10g/l | (NH₄)₂SO₄ |
| 0.4g/l | MgS0₄*7H₂O |
| 0.6g/l | KH₂PO₄ |
| 0.3mg/l | Thiamine*HCl |
| 1mg/l | Biotin |
| 2mg/l | FeSO₄ |
| 2mg/l | MnSO₄ |

adjusted to pH 7.8 with NH₄OH autoclaved (121°C, 20 min)additionally Vitamin B 12 (Hydroxycobalamine, Sigma Chemicals) is added to a final concentration of 100 µg/l.

Subsequently, one can determine the concentration of lysine that is segregated into the medium. This can be achieved by determining the amino acid concentration using HPLC on an Agilent 1100 Series LC system HPLC. A precolumn derivatisation with orthophthalaldehyde allows to quantify the formed amino acid. The separation of the amino acid mixture can be done on a Hypersil AA-column (Agilent).

The effect of using the optimized synthetic gene for lysA on the protein amount can be determined using 2D PAGE. A method how to perform 2D PAGE with proteins of *Corynebacterium glutamicum* can be found e.g. in Hermann et al. (Electrophoresis (2001), 22, 1712-1723). For the 2D PAGE analysis preferably medium without complex carbon- and nitrogen-sources is used.

It is assumed that the strains containing the optimized gene for lysA will comprise higher amounts of LysA protein compared to the wild type or parent strains that use the endogenous lysA sequence.

One may also determine the positive influence of a synthetic codon usage optimized genes by measuring the activity of lysA. This may be done by the method described in White P. J. Methods in Enzymology, 1971, 17, 140-145

### 4. Improved protein expression of lysA in C. glutamicum

In the following it is described how to increase the amount of cob(I)alamin-dependent methionine synthase (metH) by adapting the codon usage as mentioned above.

The enzyme metH is important for methionine biosynthesis. The wild type sequence of C. glutamicum metH is given as SEQ ID No. 10. The codon usage of the coding sequence of metH (SEQ ID No. 10) was determined using the Cusp function of the EMBOSS software package. The gene metH was amplified by PCR

Then, the codons corresponding to amino acid positions 53, 121 and position 154 were altered from G residues to C residues using established mutagenesis methods (Quikchange kit, Stratagene La Jolla USA) resulting in altered codons which still coded for glycine amino acids in the final protein metH (SEQ ID No. 11). The gene sequences of metH and the metH mutated amplified by PCR and fused with the sequence of the P_{GroEs} promotor as described in WO 2005059143. The resulting genes were then cloned into the vector pCLIK5 MCS yielding the vector pCLIK5 MCS PGroES metH. In this vector the metH unmutated or metH mutated are transcribed under the control of the GroES promotor and are therefore expressed to significant levels in C. glutamicum as described in WO 2005059143. As a negative control, empty vector was used. In the case of the codon optimized metH gene the same vector was used as in the case of the normal form of metH

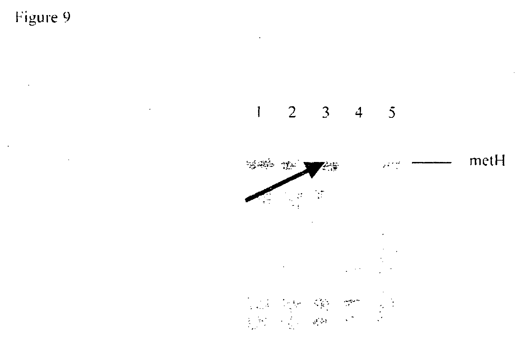

The genes were expressed in *C*. *glutamicum* as described in WO2007011845. It was found that strains expressing the mutated metH gene did show an improved and increased amount of metH protein as indicated by a gel band with increased staining and thickness (see Figure 9).

## Claims

1. Method of increasing the amount of at least one endogenous polypeptide in *C*. *glutamicum,* comprising the step of expressing in *C*. *glutamicum* a modified nucleotide sequence encoding for said at least one polypeptide, wherein the codon usage of the modified nucleotide sequence is adjusted to the codon usage of abundant proteins of *C*. *glutamicum,* wherein said modified nucleotide sequence is derived from a different starting nucleotide sequence and wherein the modified and starting nucleotide sequence encode for the same amino acid sequence and wherein at least one, some or all codons of said modified nucleotide sequence are selected from the codon usage of Table 3.

2. Method of according to claim 1, wherein at least one, some or all codons of said modified nucleotide sequence use GUU for valine, GCU for alanine, GAC for aspartic acid, GAG for glutamic acid and AUG for methionine if AUG is the start codon.

3. A recombinant modified nucleotide sequence encoding for an endogenous polypeptide of *C*. *glutamicum* which allows for increased expression of said endogenous polypeptide in *C*. *glutamicum,* wherein the codon usage of the modified nucleotide sequence is adjusted to the codon usage of abundant proteins of *C*. *glutamicum,* wherein said modified nucleotide sequence is derived from a different starting nucleotide sequence and wherein the modified and starting nucleotide sequence encode for the same amino acid sequence and wherein at least one, some or all codons of said modified nucleotide sequence for each amino acid are selected from the codon usage of Table 3.

## Patentansprüche

1. Verfahren zum Erhöhen der Menge an mindestens einem endogenen Polypeptid in *C. glutamicum,* das den folgenden Schritt umfasst: Exprimieren einer modifizierten Nukleotidsequenz, die für das mindestens eine Polypeptid codiert, in *C. glutamicum,* wobei die Codonverwendung der modifizierten Nukleotidsequenz an die Codonverwendung von abundanten Proteinen von *C. glutamicum* angepasst ist, wobei sich die modifizierte Nukleotidsequenz von einer unterschiedlichen Ausgangsnukleotidsequenz ableitet und wobei die modifizierte Nukleotidsequenz und die Ausgangsnukleotidsequenz für dieselbe Aminosäuresequenz codieren und wobei mindestens eines, manche oder alle Codons der modifizierten Nukleotidsequenz aus der Codonverwendung gemäß Tabelle 3 ausgewählt sind.

2. Verfahren nach Anspruch 1, wobei, wenn AUG das Start-Codon ist, mindestens eines, manche oder alle Codons der modifizierten Nukleotidsequenz GUU für Valin, GCU für Alanin, GAC für Asparaginsäure, GAG für Glutaminsäure und AUG für Methionin verwenden.

3. Rekombinante modifizierte Nukleotidsequenz, die für ein endogenes Polypeptid von *C. glutamicum* codiert, was die erhöhte Expression des endogenen Polypeptids in *C. glutamicum* gestattet, wobei die Codonverwendung der modifizierten Nukleotidsequenz an die Codonverwendung von abundanten Proteinen von *C. glutamicum* angepasst ist, wobei sich die modifizierte Nukleotidsequenz von einer unterschiedlichen Ausgangsnukleotidsequenz ableitet und wobei die modifizierte Nukleotidsequenz und die Ausgangsnukleotidsequenz für dieselbe Aminosäuresequenz codieren und wobei mindestens eines, manche oder alle Codons der modifizierten Nukleotidsequenz für jede Aminosäure aus der Codonverwendung gemäß Tabelle 3 ausgewählt sind.

## Revendications

1. Procédé d'augmentation de la quantité d'au moins un polypeptide endogène dans *C*. *glutamicum,* comprenant l'étape d'expression dans *C*. *glutamicum* d'une séquence de nucléotides modifiée codant pour ledit au moins un polypeptide, dans lequel l'usage de codon de la séquence de nucléotides modifiée est adapté à l'usage de codon de protéines abondantes de *C*. *glutamicum,* où ladite séquence de nucléotides modifiée est dérivée d'une séquence de nucléotides de départ différente et dans lequel les séquences de nucléotides modifiée et de départ codent pour la même séquence d'acides aminés et dans lequel au moins un, certains ou l'ensemble des codons de ladite séquence de nucléotides modifiée sont choisis parmi l'usage de codon du tableau 3.

2. Procédé selon la revendication 1, dans lequel au moins un, certains ou l'ensemble des codons de ladite séquence de nucléotides modifiée utilisent GUU pour la valine, GCU pour l'alanine, GAC pour l'acide aspartique, GAG pour l'acide glutamique et AUG pour la méthionine si AUG est le codon d'initiation.

3. Séquence de nucléotides modifiée recombinante codant pour un polypeptide endogène de *C*. *glutamicum* qui permet l'expression augmentée dudit polypeptide endogène dans *C*. *glutamicum,* où l'usage de codon de la séquence de nucléotides modifiée est adaptée à l'usage de codon de protéines abondantes de *C*. *glutamicum,* où ladite séquence de nucléotides modifiée est dérivée d'une séquence de nucléotides de départ différente et où la séquence de nucléotides modifiée et de départ codent pour la même séquence d'acides aminés et où au moins un, certains ou l'ensemble de codons de ladite séquence de nucléotides modifiée pour chaque acide aminé sont choisis parmi l'usage de codon du tableau 3.
